# EUROPEAN PATENT APPLICATION

(11) **EP 3 543 255 A1**
(43) Date of publication of application: **25.09.2019**
(21) Application number: 18163074.0
(22) Date of filing: 21.03.2018
(51) Int. Cl.: C07K 16/04, C07K 16/12, A61K 39/00, A61L 27/52

(54) **TREATMENT OF STAPHYLOCUCCUS RELATED DISEASES**

(71) Applicant: Förster, Beatrix, 3273GX Bilthove (NL); Winter, Gerhard, 82377 Penzberg (DE); Geh, Katharina, 86459 Gessertshausen (DE)
(72) Inventor: Förster, Beatrix, 3273GX Bilthove (NL); Winter, Gerhard, 82377 Penzberg (DE); Geh, Katharina, 86459 Gessertshausen (DE)
(74) Representative: Banse & Steglich Patentanwälte PartmbB

(57) **Abstract**

The present invention relates to antibodies for treating or preventing infections of *Staphylococcus* genus bacteria and/or *Staphylococcus* genus bacteria-related diseases. Especially, the invention relates to *S. intermedius* group bacteria infections and diseases. Furthermore, the invention relates to respective pharmaceutical compositions and methods of manufacturing a medicament comprising anti-*S. aureus* alpha-hemolysin protein antibodies.

## Description

### Field of invention

The present invention relates to antibodies for treating or preventing infections of *Staphylococcus* genus bacteria and/or *Staphylococcus* genus bacteria-related diseases. Especially, the invention relates to *Staphylococcus intermedius* group bacterial infections and diseases. Furthermore, the invention relates to respective pharmaceutical compositions and methods of manufacturing a medicament.

### Background

*Staphylococcus pseudintermedius* (*S. pseudintermedius*) is a gram- und coagulase-positive bacterium. It is a skin and mucous membrane commensal in the dog and the most frequent bacterial pathogen isolated from clinical canine specimens.

*S. pseudintermedius* belongs to the *Staphylococcus intermedius* (*S. intermedius*) group (SIG), which comprises the three distinct species, *S*. *intermedius, S. pseudintermedius and S. delphini.* The SIG group belongs to the *Staohyloccus* genus.

*S. pseudintermedius* is an opportunistic pathogen which resides in part in the normal microbiome of most dogs. It does not cause any disease, unless the resistance of the host is lowered and the skin barrier is altered by predisposing factors, such as atopic dermatitis, medical and surgical procedures and/or immunosuppressive disorders or physical injury by scratching and biting. However, in coincidence with one of these factors, *S. pseudintermedius* may be causative for a number of diseases, of which pyoderma is the most frequent disease. Canine pyoderma is observed in clinical manifestations such as superficial folliculitis, deep folliculitis and furunculosis, bacterial overgrowth syndrome, pyotraumatic folliculitis/furunculosis ("hotspots" with satellite lesions), intertrigo (skin fold dermatitis), mucocutaneous pyoderma, bullous impetigo acral lick granuloma and folliculitis/furunculosis, nasal folliculitis and furunculosis, chin acne (muzzle folliculitis and furunculosis), staphylococcal necrotising fasciitis and Staphylococcal toxic shock-like syndrome (Schmidt, 2010). Clinical signs include pruritus, erythema, pustules and papules, follicles, ulcerated plaques, nodules, haemorrhagic bullae comedones, furuncles, erosions, ulcers, oedema, epidermal collarettes and crusted erosions. These lesions may be painful and the animal may also show signs of systemic illness, such as fever and lymphadenopathy. Further symptoms include lichenification, hyperpigmentation and scale.

In addition to pyoderma, *S*. *pseudintermedius* is also frequently isolated from canine ear and wound infections, may cause gingivitis, hepatitis, respiratory infections, arthritis, peritonitis, septicaemia and can be a complicating factor in immunomodulatory-responsive lymphocytic-plasmacytric pododermatitis (Pomba et al., 2017). Also, *S. pseudintermedius* is the species most commonly isolated from canine urinary tract infections. It has also been identified as the causative agent of an infection following a joint prosthesis in a dog and as a fatal cause of canine necrotizing fasciitis.

*S. pseudintermedius* infections are commonly treated by topical or systemic antibiotic therapy. However, it has been shown that the antibiotic sensitivity of SIG isolates varies, which poses a problem for the choice of the right antibiotic. Commonly used antibiotics include varycefadroxil, cefalexin, clavulanateamoxicillin, clindamycin, lincomycin, tetracyclines and sulfonamides as first line antibiotics, cefovecin, cefpodoxime, difloxacin, enrofloxacin, marbofloxacin, orbifloxacin, fluoroquinolones and pradofloxacin as second line antibiotics and aminoglycosides, azithromycin, ceftazidime, chloramphenicol, clarithromycin, florphenicol, imipenem, phosphomycin, piperacillin, rifampin, tiamphenicol and ticarcillin as third line antibiotics (Beco et al., 2013).

However, the effectiveness of antibiotic therapy is hampered by the occurrence of antibiotic resistant or even multi-resistant *S. pseudintermedius* group bacterial strains. Methicillin-resistant (MRSP) or even multi-resistant strains which may be resistant to at least five antimicrobial classes have been identified (Pomba et al., 2017; Loeffler et al., 2007).

Antibiotic resistant *S. pseudintermedius* not only poses a major problem for veterinary healthcare, but also represents a health risk for humans, due to dog-to-human transmission of bacterial infection which have frequently been observed. *S. pseudintermedius* infections in humans were formed causative for endocarditis, local lesions, bacteremia, brain abscesses or pneumonia, among others (Lozano et al., 2017).

Another pathogenic bacterial species from the *Staphylococcus* genus, for which antibiotic resistance is frequently observed, is *Staphylococcus aureus* (*S. aureus*). Methicillin-resistant *S. aureus* (MRSA) is a significant cause for wound infections in companion animals (Walther et al., 2017) and causes hospital and community-acquired infections in humans (Pomba et al., 2017).

In view of the increasing morbidity caused by MRSA, several vaccines against *S. aureus* toxin antigens were evaluated to combat *S. aureus* infections. One toxin that has been tested as antigen for immunizing is the *S. aureus* alpha-hemolysin (Hla; also known as alpha toxin). Alpha-hemolysin is a membrane-damaging exoprotein with hemolytic activity that oligomerizes to form transmembrane pores. Hla forms pores in lymphocytes, macrophages, alveolar epithelial cells, pulmonary endothelium and erythrocytes (Wardenburg und Schneewind, 2008). The hemolytic activity of alpha-hemolysin is completely diminished by substituting histidine 35 with leucine (H35L). Mutant forms H48L, H144L and H259L of alpha-hemolysin exhibit a reduced hemolytic activity (Menzies and Kernodle, 1994). Active immunization with the H35L mutant form of Hla (H35L) has been shown to generate antigen-specific immunoglobulin G responses and afforded protection against staphylococcal pneumonia in a mouse model. Furthermore, passive transfer of Hla-specific antibodies protected naive animals against *S. aureus* challenge (Wadenburg & Schneewind, 2008). However, no effect on other *Staphylococcus* species has been shown. Furthermore, a vaccine approach is not suitable for the treatment of established infections, but serves as a protective measure only. Passive transfer of antibodies requires large amounts of antibodies and furthermore requires systemic administration. Thus, passive immunization is a costly therapy and cannot be easily administered. With regard to passive transfer or and are therapy based on the administration of polyclonal anti-alpha-hemolysin antibody composition, it is a major disadvantage that their respective antibody compositions can only be obtained from immunized with the respective antigen or from monoclonal hybridomas. Therefore, these antibodies cannot easily be obtained and are relatively costly, which is especially relevant for the treatment of animals, where treatment costs are usually not covered by a health insurance.

Zakour and colleagues reported the first whole-genome sequence of an *S*. *pseudintermedius* strain (Zakour et al., 2011). While the genome encodes predicted homologues of beta-hemolysin delta-hemolysin (hemolysin III) and leukotoxin Luk-I, no homologue for alpha-hemolysin or other leukotoxins is dislosed.

Some vaccines for the prevention of *S. intermedius* group bacterial infections are known in the art.

WO 2010/094101 A1 discloses a vaccine composition for the prevention of pyoderma in dogs. The vaccine composition comprises virulence factors of *S. intermedius,* such as protein A, coagulases, hemolysins, enterotoxins and exofolative toxins. While type beta and delta hemolysins are disclosed, no *S. intermedius* alpha-hemolysin, LukD, LukE, LukF or HlgB are disclosed as antigen. While the disclosed passive vaccine compositions might be suitable to prevent pyoderma in dogs, passive vaccine compositions are not suitable for treating established pyoderma in dogs.

U.S. 2012/0282289 A1 discloses the use of several *S*. *pseudintermedius* surface antigens for use in vaccine compositions. Mice vaccinated with domains from SpsD (a protein with homology to Fnbp protein homologue of *S. aureus* Mu50), SpsL (another protein with homology to Fnbp protein homologue of *S. aureus* Mu50) and SpsA (a protein with homology to LPXTG cell-wall surface anchor family protein of *S. aureus* COL) showed reduced lesions after challenge with *S*. *pseudintermedius.* However, only the prevention of an infection, but no treatment of an established pyoderma is disclosed.

In summary, *Staphylococcus* infections, especially infections related to *S. intermedius* group bacteria and *S. aureus,* represent a risk to animal and human health. The risk is further increased by the development of antibiotic resistance in these bacteria. The present non-antibiotic treatments are limited to active or passive vaccination for the prevention of *Staphylococcus* infections. Furthermore, no non-antibiotic treatment is available that can be conveniently administered by a pet owner or human patient on a regular basis for the local treatment of *Staphylococcus* infections.

### Problem underlying the invention

In view of the prior art, it was the general problem underlying the present invention, to provide pharmaceutically active agents, compositions, methods and uses to overcome the above-mentioned disadvantages of the prior art. Especially, agents, compositions and methods suitable for providing a non-antibiotic treatment of established *Staphylococcus-*related infections and diseases should be provided. Specifically, agents, compositions and methods for the treatment or prevention of *S. intermedius* group bacteria-related infections and diseases should be provided. Especially, the agents and compositions should be easily obtainable at relatively low costs. Furthermore, the agents and compositions should be conveniently administrable and safe.

In a further aspect, suitable formulations for administering the agents and compositions should be provided.

### Disclosure of the invention

Surprisingly, it was found that the problem underlying the invention is solved by the antibodies, compositions and methods according to the claims. Further embodiments of the invention are outlined throughout the description.

In a first aspect, the invention relates to at least one naturally occurring antibody for use in the treatment or the prevention of infections of *Staphylococcus* genus bacteria and/or a *Staphylococcus* genus bacteria-related disease. Preferably, the at least one antibody is a polyclonal antibody.

Within the context of the present invention, a "naturally occurring antibody" is an antibody present in a subject under natural conditions. Within the context of the present invention, "natural conditions" refer to conditions wherein a subject is not intentionally immunised with an antigen for producing antibodies to this antigen. Especially, the subject is not immunized with isolated *Staphylococcus* genus bacteria, isolated part thereof, an isolated *Staphylococcus* genus bacteria protein, and/or isolated part or fraction thereof. Especially the subject is not immunised with a recombinant bacterial protein, and/or part thereof. An isolated part of *Staphylococcus* genus bacterium may be a composition which comprises enriched or isolated parts or fractions of a bacterial cell, such as organelles, cytosol, or cell/organelle membrane fractions.

Thus, the antibody according to the present invention may be obtained from a naturally occurring antibody source. According to the present invention, a "naturally occurring antibody source" may be an animal not intentionally immunized with isolated *Staphylococcus* genus bacteria, isolated part thereof, an isolated *Staphylococcus* genus bacteria protein, and/or isolated parts or fractions thereof as described above.

According to the present invention, the *Staphylococcus* bacterial genus at least comprises the *S. aureus* group; *S*. *auricularis* group, *S*. *auricularis, S. carnosus* group, *S*. *epidermidis* group, *S*. *haemolyticus* group, *S*. *hyicus-group, S*. *intermedius, S. lugdunensis* group, *S. saprophyticus* group, *S. sciuri* group, *S. simulans* group, *S. warneri* group.

According to the present invention, the *S. aureus* group at least comprises *S. argenteus, S. aureus, S. schweitzeri* and *S. simiae.* The *S. auricularis* group at least comprises *S. auricularis.* The *S. carnosus* group at least comprises *S. carnosus, S. condimenti, S. massiliensis, S. piscifermentans, S. simulans.* The *S. epidermidis* group at least comprises *S. capitis, S. caprae, S. epidermidis, S. saccharolyticus.* The *S. haemolyticus* group at least comprises *S. devriesei, S. haemolyticus, S. hominis.* The *S. hyicus*-group at least comprises *S. agnetis, S. chromogenes, S. felis, S. hyicus, S. lutrae, S. microti, S. muscae, S. rostri, S. schleiferi. The S. lugdunensis* group at least comprises *S. lugdunensis.* The *S. saprophyticus* group at least comprises *S. arlettae, S. cohnii, S. equorum, S. gallinarum, S. kloosii, S. leei, S. nepalensis, S. saprophyticus, S*. *succinus, S. xylosus.* The *S. sciuri* group at least comprises *S. fleurettii, S. lentus, S sciuri, S. stepanovicii, S. vitulinus. S. simulans* group at least comprises *S. simulans.* The *S. warneri* group at least comprises *S. pasteuri, S. warneri.* The *S. intermedius* group (SIG) ate least comprises or consists of the species *S. intermedius, S. pseudintermedius* and S. delphini.

In a preferred embodiment, the *Staphylococcus* bacterial is from the *S. aureus* or *S. intermedius* group of bacteria, most preferably from the S. *intermedius* group of bacteria.

Within the context of the present invention, "treating or preventing" infections of *Staphylococcus* genus bacterial infections or *Staphylococcus* genus bacteria-related diseases, relates to the application of a compound or composition, specifically the polyclonal antibodies and compositions described herein, for (a) preventing the infection, disease or symptom thereof from occurring in a subject which may be predisposed to and/or may acquire the infection, disease or symptom thereof, but has not yet been diagnosed as having it; (b) inhibiting the infection or disease symptoms, i.e. arresting its development; or (c) relieving or eliminating the infection or disease symptoms, i.e. causing regression of the infection, disease or symptoms thereof.

Within the context of the present invention, *"Staphylococcus* genus bacteria-related diseases" are pathological conditions which are caused directly or indirectly by a bacterium from the *Staphylococcus* genus or a constituent or product of a *Staphylococcus* genus bacterium, especially by a toxic protein expressed by a *Staphylococcus* genus bacterium. The *Staphylococcus* genus bacteria infection or related disease may be a systemic or a localized infection or disease. Preferably, the condition is caused by *S. aureus* or *S. intermedius* group of bacteria, most preferably from the *S. intermedius* group of bacteria.

It was surprisingly found that a polyclonal antibody derived from cows that we were not intentionally immunized with isolated *S. pseudintermedius* bacteria, isolated part thereof, an isolated *S. pseudintermedius* protein, and/or isolated part or fraction thereof binds to the surface of drug-resistant S. pseudintermedius bacteria.

As shown in Example 6, the polyclonal antibody derived from cows that were not intentionally immunized with isolated *Staphylococcus* genus bacteria, isolated part thereof, an isolated *Staphylococcus* genus bacteria protein, and/or isolated part or fraction thereof, as described above, inhibited *S. pseudintermedius* induced red blood cell lysis to at least the same extend as polyclonal antibody obtained from a cow immunized with isolated recombinant Hla.

Furthermore, Example 4 indicates that surprisingly polyclonal antibodies obtained from a cow immunized with isolated recombinant Hla were not only immunoreactive with *S. aureus* alpha-hemolysin, but also with *S. aureus.* beta-hemolysin, LukD, LukE, LukF, and HlgB. *S*. *aureus.* Mature alpha-hemolysin is a polypeptide corresponding to amino acids 27 to 319 of the UniProtKB/Swiss-Prot Database Entry No. P09616.2. S. aureus. beta-hemolysin is a polypeptide corresponding to amino acids GenBank Accession No. ABS19574. *S. aureus* LukD refers to *S. aureus* leukotoxin, LukD corresponding to GenBank Accession No. BBA24454.1. LukE refers to S. aureus leukocidin LukE corresponding to Accession No. BBA24454.1. LukF refers to S. aureus leukocidin LukF corresponding to amino acids 25 to 325 of GeneBank Accession No. CAA51252. HlgB refers to *S. aureus* gamma-hemolysin component B corresponding to amino acids 26 to 325 of GenBank Accession No. AAA26639.

Thus, in an alternative embodiment, the present invention relates to a polyclonal antibody immunoreactive with *S. aureus.* alpha-hemolysin, beta-hemolysin, LukD, LukE, LukF, and/or HlgB for use in the treatment or the prevention of infections of *Staphylococcus* genus bacteria and/or a *Staphylococcus* genus bacteria-related disease.

It could furthermore surprisingly be shown in Example 9 below that a pharmaceutical composition comprising a polyclonal antibody according to the invention has a therapeutic effect for the treatment of pyoderma in dogs. Thus, naturally occurring polyclonal antibodies according to the invention can advantageously and surprisingly be used as a non-antibiotic active agent in the treatment or prevention of infections of *Staphylococcus* genus bacteria and/or *Staphylococcus* genus bacteria-related diseases, especially in the treatment or prevention of infections of infections of *S. intermedius* group bacteria and/or *S. intermedius* group bacteria-related diseases.

Surprisingly, it seems that already the natural bacterial colonialization of animals, such as for example cows, with bacteria from the *Staphylococcus* genus induces polyclonal antibody titers that enable the isolation of polyclonal antibodies suitable for a therapeutic application.

Thus, the inventors surprisingly show that an immunization of animals with an isolated antigen is not required to obtain polyclonal antibodies suitable for a therapeutic application.

Within the context of the present invention, a "polyclonal antibody" is a population of different antibody molecules which are secreted by different B-Cell lineages. A polyclonal antibody may preferably be capable of binding several different specific antibody epitopes within a certain protein. The different antibody molecules comprised in the polyclonal antibody may have different variable regions, in particular in the CDR1, CDR2 and CDR3 regions.

The systemic infection or disease according to the present invention is an infection or disease that is in the bloodstream or affects the whole body. A systemic *S. intermedius* group bacteria infection or related disease may for example be arthritis, septicemia, bacteremia or staphylococcal toxic shock-like syndrome.

The localized infection or disease according to the present invention affects only a body part or an organ. A localized *S. intermedius* group bacterial infection or related disease may be an ear or wound infection gingivitis, respiratory infection, e.g. pneumonia; peritonitis or pyoderma. In a preferred embodiment, the localized infection is a skin, mucosa or soft tissue infection or disease.

In a preferred embodiment, the infection or disease treated according to the present invention is pyoderma. The pyoderma may be superficial folliculitis, a deep folliculitis and furunculosis, a bacterial overgrowth syndrome, pyotraumatic folliculitis and/or furunculosis ("hotspots" with satellite lesions), intertrigo (skin fold dermatitis), mucocutaneous pyoderma, bullous impetigo, acral lick granuloma, acral lick folliculitis and/or furunculosis, nasal folliculitis and furunculosis, a chin acne (muzzle folliculitis and furunculosis) and/or staphylococcal necrotising fasciitis. The treated symptoms of pyoderma may be pruritus, erythema, pustules and papules, follicles, ulcerated plaques, furuncles, nodules, haemorrhagic bullae comedones, pustules, papules, furuncles, erosions, ulcers, oedema, epidermal collarettes, crusted erosions, lichenification, hyperpigmentation and scale.

According to the present invention, mammals and birds may be treated with the polyclonal antibody described herein. Preferably, the mammal is a human or a domestic animal. Preferably, the domestic animal is a dog, cat or horse. Most preferably, a human or dog is treated.

The different species of the *Staphylococcus* genus, especially the different species of the S. *intermedius* group of bacteria may be discriminated by different methods. Discriminatory phenotypic tests for differentiating *S. pseudintermedius* from other *Staphylococcus* species isolated from dogs may include a positive coagulase phenotype, a negative acetoin production phenotype, a negative pyrrolidonyl arylamidase phenotype, a positive beta-galactosidase phenotype, a negative polymyxin B resistance and delayed D-mannitol acidification according to Banoehr et al., 2017. *S. intermedius* may be differentiated from S. *pseudintermedius* and S. delphini phenotypically by a negative argenin hydrolase reaction, acid production from positive B-gentobiose test in aerobic conditions and positive anaerobically D-mannitol test (Banoehr at al., 2007). More accurate methods for the identification of the *Staphylococcus* species may be conducted based on molecular mechanisms such as polymerase chain-restriction fragment length polymorphism (PCR-RFLP) (Banoehr et al., 2017) as well as bacterial typing by MALDI-TOF Mass Spectrometry. PCR-RFLP also allows the discrimination of *S. pseudintermedius* from the closely related members of the *S. intermedius* group, for example S. delphini, and other important staphylococcal pathogens of humans and dogs (Banoehr et al., 2007).

In a preferred embodiment of the present invention, the *S. intermedius* group bacterium is *S. pseudintermedius.*

In another preferred embodiment of the present invention, the *Staphylococcus* genus bacterium, especially the *S. intermedius* group bacterium is a drug-resistant strain, preferably an antibiotic-resistant strain. A drug-resistant strain is a strain which has a reduced or no susceptibility to certain drugs. In a preferred embodiment, the drug resistant strain is an antibiotic resistant strain. In antibiotic resistant bacterial strains, the inhibition of bacterial proliferation is not inhibited by the respective antibiotic or inhibition of proliferation is only observed at higher antibiotic concentrations as compared to a non-antibiotic resistant bacterial strain. Antibiotic resistance may generally be tested in an agar diffusion assay according to DIN 58940. The antibiotic resistant strain may preferably be a methicillin-resistant (MRSP) strain. The methicillin-resistant strain may be a macA gene-positive strain. The macA gene confers resistance to beta-lactam antibiotics. The presence of the macA gene in a strain might be tested by PCR with macA-specific primers (Loeffler et al., 2007). Thus the strain might be a strain resistant against a beta-lactam antibiotic. The beta-lactam may be a penicillin, a Penem, a Carbapenem, a Cephem such as a cephalosporin or a cephamycin; a monobactam or a β-lactamase inhibitor. The penicilin may be a benzylpenicillin, benzathine, benzylpenicillin, procaine benzylpenicillin, benzylpenicillin, phenoxymethylpenicillin , propicillin, pheneticillin, azidocillin, clometocillin, penamecillin, , amoxicillin, an ampicillin such as pivampicillin, hetacillin, bacampicillin, metampicillin, talampicillin; epicillin, ticarcillin, carbenicillin/carindacillin, temocillin, piperacillin, azlocillin, mezlocillin, mecillinam, sulbenicillin, a sloxacillin such as dicloxacillin or flucloxacillin, oxacillin, nafcillin or methicillin. The penem may be a faropenem or ritipenem. The Carbapenem may be ertapenem, an antipseudomonal such as doripenem, imipenem or meropenem, biapenem or panipenem. The cephalosporin or a cephamycin may be cefazolin, cefalexin, cefadroxil, cefapirin, cefazedone, cefazaflur, cefradine, cefroxadine, ceftezole, cefaloglycin, cefacetrile, cefalonium, cefaloridine, cefalotin, cefatrizine, cefaclor, cefotetan, cefoxitin, cefprozil, cefuroxime, cefuroxime axetil, cefamandole, cefminox, cefonicid, ceforanide, cefotiam, cefbuperazone, cefuzonam, cefmetazole, carbacephem, cefixime, ceftriaxone, an antipseudomonal such as ceftazidime or cefoperazone; cefdinir cefcapene, cefdaloxime, ceftizoxime, cefmenoxime, cefotaxime, cefpiramide, cefpodoxime, ceftibuten, cefditoren, cefetamet, cefodizime, cefpimizole, cefsulodin, cefteram, ceftiolene, an oxacephem such as flomoxef or latamoxef; ceftiofur, cefquinome, ceftaroline fosamil, ceftolozane, ceftobiprole, cefepime, cefozopran, cefpirome, or cefquinome. The monobactam may be aztreonam, tigemonam, carumonam or nocardicin A. The beta lactamase inhibitor may be sulbactam, tazobactam, clavulanic acid (amoxycilin), avibactam or vaborbactam. The resistance might also be against a fluoro-quinolone ciprofloxacin antibiotic such as ofloxacinenoxacin, fleroxacin, lomefloxacin, nadifloxacin, norfloxacin, pefloxacin, rufloxacin, levofloxacin, balofloxacin, grepafloxacin, pazufloxacin, sparfloxacin, temafloxacin, tosufloxacin, besifloxacin, gatifloxacin, finafloxacin, gemifloxacin, moxifloxacin, clinafloxacin, garenoxacin, prulifloxacin, sitafloxacin, trovafloxa-cin, alatrofloxacin, danofloxacin, difloxacin, enrofloxacin, ibafloxacin, marbofloxacin, orbifloxacin, pradofloxacin, or sarafloxacin.

Preferably the drug resistant strain is a strain resistant against methicilin, cefalexin, enrofloxacin, penicilin, ampicilin, clavulanic acid (amoxycilin), oxacillin, cefalexin, cephalothin, clindamycin, erythromycin, gentamycin, refampicin, tetracyclin, trimethoprim, kanamycin or ciproflaxin. In a further preferred embodiment, the strain is a multi-resistant strain, resistant against at least two antibiotics, more preferably against at least three, at least five or at least six antibiotics. A methicillin-resistant S pseudintermedius strain is commonly known as MRSP.

In a preferred embodiment, the drug resistant *S pseudintermedius* strain may be a strain selected from at least one of the strains C8187, C8386, C8188, C8477, C8189, C8478, or C8470 as disclosed by Lozano et al., 2017; or a strain selected from 69687, 69876, HH15, GL 119A, GL151A, 23929, 1726, BNG1, BNG3, GL117B, GL118B, 463949, HKU10-03, E140, or ED99 as disclosed by McCarthy et al., 2015; or any of the strains 23939, 69687, MRSPHH15, BNG I disclosed in Example 7.

In another preferred embodiment, the drug resistant strain is an *S. aureus* strain. The drug resistant *S. aureus* strain may for example be E2125, HPV107, F-182, Mu3, Mu50, Mu50, 2947, 406, 12478, NYBK246, HFH-29994, HFH-29753, HFH-30106, HFH-30008, HFH-29744, HFH-30123, HFH-30172, HFH-30239, HIP 10787, MRSA252, 96:281, 148-99, 18626, 107-03, GA201, 1063, CPS22, 308118L, 328, 1217, HUSA304, HSJ216, FPR3757, HFH-29568, HFH-30364, HFH-30676, HFH-30137, HFH-30102, HFH-30493, HFH-30626, HFH-33798, HFH-31076, MW2†, TCH1516, 94:1013, 182-99, 00:50, 0-25-4, 0-25-37, 1-1-81, 1-1-493, GA217, GA229, 7031, 510-04, 27-05, CA46, N4151, CL604, HFH-30032, 8-03, B8-31, HDE288, M10/0061, and/or M10/0148.

It was surprisingly found that a naturally occurring antibody according to the present invention binds to antibiotic-resistant *S. intermedius* strains. Thus, naturally occurring polyclonal antibodies according to the present invention can advantageously be used as a non-antibiotic active agent for treating or preventing infections of antibiotic-resistant *S. intermedius* group bacteria and/or antibiotic-resistant *S. intermedius* group bacteria-related diseases. The use of antibodies as active agent is generally advantageous for the treatment of infections of antibiotic-resistant *S. pseudintermedius* strains, since they are non-antibiotic active agents, thus active agents which rely on a different mode of action than antibiotics. Moreover, the use of polyclonal antibodies may be advantageous over monoclonal antibodies, since polyclonal antibodies usually bind to several sides within the antigen. Thus, the development of a resistance against the antibody based on a single mutation, which frequently appears in bacteria genomes, is substantially reduced. Furthermore, the use of monoclonal and polyclonal antibodies as active agents is advantageous for the treatment of subjects which are allergic to certain antibiotics.

The antibody according to the invention may bind to at least one epitope selected from epitopes comprising the amino acid sequence KIGGLIG, ATKQQSN, KKILVIRTK, IDVIYERV, KAADNFLDP and/or DSDINIK. Most preferably the antibody may bind to at least the epitope comprising the amino acid sequence KIGGLIG.

Preferably, a polyclonal antibody binds to at least two, at least three, at least four or at least five epitopes.

The antibody may be an immunoglobulin selected from the immunoglobulin classes IgG, IgM, IgA, IgD, IgE and IgY. The IgG may be selected from at least one of the subclasses IgG1, IgG2, IgG3, IgG4, IgA1, IgA2. Preferably, the anti-S. *aureus* Hla antibody comprises at least one immunoglobulin from the IgG class.

Preferably the antibody is an immunoglobulin G that binds to at least one epitope selected from epitopes comprising the amino acid sequence KIGGLIG, ATKQQSN, KKILVIRTK, and/or IDVIYERV. The antibody may also bind to several epitopes, preferably KIGGLIG and ATKQQSN.

In another preferred embodiment the antibody is an immunoglobulin A or M that binds to at least one epitope selected from epitopes comprising the amino acid sequence IDVIYERV, KAADNFLDP.

The antibody may be an intact tetrameric antibody. In further embodiments, the antibody may be an antibody-binding fragment of an antibody. Antibody fragments include Fab, Fab', F(ab')2 and Fv fragments and single-chain antibodies (e.g. scFv). Fab fragments may be obtained by papain digestion.

The antibody may be a chicken antibody or a mammalian antibody. The mammalian antibody may be a human, mouse, rat, sheep, dog, goat, rabbit, equine, llama or bovine antibody. In a preferred embodiment, the antibody is a bovine antibody. Within the present invention, the described species' origin of the antibody may define an antibody that was expressed in an animal of the respective species.

The polyclonal antibody according to the invention may be obtained by isolating the polyclonal antibody from the body fluids of the animal. Preferably, the antibody may be isolated from blood, preferably from blood serum, milk or colostrum. Colostrum is the first lacteal fluid that is produced by a female animal following birth of a young. In comparison to milk, which is produced later, colostrum comprises high amounts of immunoglobulins, especially IgG. Thus, the use of colostrum is especially advantageous. Thus, the antibody according to the present invention may be an isolated antibody.

In a preferred embodiment, the polyclonal antibody is obtained from bovine milk or colostrum.

The isolation of the polyclonal antibody from colostrum or milk may preferably comprise the enrichment of IgG by membrane filtration according to the methods disclosed by Piott et al., 2004, to obtain an enriched composition comprising the antibody described herein.

In a preferred embodiment, the anti-S. *aureus* Hla antibody may be purified or partially purified by chromatography techniques. Chromatography techniques for purifying antibodies are well-known in the art. The chromatography technique may, for example, be a cationic exchange chromatography, a hydrophobic interaction chromatography, an affinity chromatography or a combination of these techniques. In a preferred embodiment, the antibody is purified in a first step by cation exchange chromatography, preferably at neutral pH. Preferably, SP sepharose or CM sepharose may be used as cation exchange material. In an alternative preferred embodiment, a multimodal cationic chromatography material, for example Capto MMC (GE Health Care, Bio-Sciences, Pittsburgh, USA) may be used. At neutral pH, impurities, for example whey proteins, lactoperoxidase or lactoferrin, may be separated from the antibody by binding to cation exchange material, whereas the antibody does not substantially bind to the cation exchange material.

The antibody may be further purified by hydrophobic charge induction chromatography/HCIC at neutral pH. Preferably, the hydrophic charge induction chromatography is performed on a 4-mercaptoethyl-pyridine (MEP) chromatography resin, for example MEP Hyper CEL (Pall Corporation, New York, USA). At neutral pH, the antibody is bound to the hydrophobic charge induction chromatography resin whereas other proteins may be separated by non-binding to the respective chromatography material. The antibody may be eluted at an acidic pH.

In a preferred embodiment, the antibody described herein may be purified by performing hydrophobic charge induction chromatography/HCIC after a chromatography step involving cationic exchange chromatography.

It was surprisingly found that an antibody according to the invention enables the treatment of *Staphylococcus* genus bacteria-related diseases, like pyoderma, even when the antibody is topically administered.

Thus, in a further aspect, the invention relates to an antibody as described herein for treating or preventing infections of *Staphylococcus* genus bacteria and *Staphylococcus* genus bacteria-related diseases by topical administration of the antibody described herein. Preferably, the infection or disease is an *S. aureus* or *S. intermedius* group bacteria infection or related disease. More preferably, the infection or disease is an *S. pseudintermedius* infection or related disease as described above. Most preferably, the disease is pyoderma.

The infection or disease may be treated or prevented by administering the antibody and/or the pharmaceutical composition as described herein. Most preferably, the infection or disease is treated by topically administering the antibody in a hydrogel as described below.

In contrast to the prevention of systemic *Staphylococcus* genus bacterial infections by passive immunization as known in the art, a topical treatment offers several advantages. Firstly, systemic treatments, like passive immunization, required the administration of the medication by a medical professional, such as a veterinarian or a medical doctor. In contrast thereto, a topical administration can be conveniently and repeatedly be administered by the owner of an animal or a human patient himself. Also, immunological reactions against the administered antibody, leading to side effects or diminishing the therapeutic effect, are less likely to occur upon topical administration of an antibody. Furthermore, due to the limited body surface usually treated by a topical treatment, small amounts of antibodies are required as compared to a systemic treatment.

In a further aspect, the invention relates to a pharmaceutical composition comprising an antibody as described herein, optionally together with a pharmaceutical acceptable carrier.

The pharmaceutically acceptable carrier may include agents, e.g. diluents, stabilizers, adjuvants or other types of excipients that are non-toxic to the cell or mammal to be exposed thereto at the dosages and concentrations employed. Examples of pharmaceutically acceptable carriers are well-known in the art and include phosphate-buffered saline solutions, water, emulsions, such as oil/water emulsions, creams, ointments and gels. In a preferred embodiment, the gel may be a hydrogel.

The cream, ointment, or gel may comprise at least one lubricant. The lubricant may for example be selected from cetyl esters wax, hydrogenated vegetable oil, methyl stearate, mineral oil, polyoxyethylene-polyoxypropylene copolymer, polyethylene glycol, polyvinyl alcohol, sodium lauryl sulfate, white wax, or mixtures of two or more of the above.

The cream, ointment, or gel may comprise at least one adjuvant, wherein the adjuvant may be selected from an antimicrobial agent, antioxidant, humectant or emulsifier, or mixture of two or more thereof.

In a further aspect, the present invention relates to a hydrogel. The hydrogel may comprise the antibody according to the invention.

In an alternative embodiment, the hydrogel may not comprise the antibody according to the invention, but may comprise a different antibody, especially an antibody for topical administration.

The hydrogel according to the invention may comprise water and a gelling agent. Typically, the hydrogel may comprise about 0.5% to about 5% w/w of the gelling agent. Preferably, the hydrogel comprises about 1% to about 2.5% w/w, more preferably about 1% to about 2% w/w. Most preferably, the hydrogel comprises about 1.5% w/w of the gelling agent.

The gelling agent may, for example, be sodium alginate, starch, protein, gelatin, hyaluronate, chitin, xanthan gum, arabic gum, pectin and/or their derivatives. Preferably, gelling agent is a cellulose derivative. Preferably, the cellulose derivative is methyl cellulose (MC), hydroxypropylmethyl cellulose (HPMC), sodium carboxymethyl cellulose (CMC), hydroxypropyl cellulose (HPC), hydroxyethyl cellulose (HEC).

Most preferably, the cellulose derivative is a sodium carboxymethyl cellulose (CMC). Preferably, the CMC has a degree of substitution of about 0.5 to about 1, preferably about 0.65 to about 0.9. Preferably the CMC is a high viscosity CMC. The CMC may for example be selected from Blanose 7H4F, Blanose 7HOPH, Aqualon 7H3SF, Aqualon 7M8SF, and/or Blanose 7LP (all Ashland, Kentucky, USA).

The CMC may have a molecular weight from 250 kDa to 2500 kDa, preferably from 400 kDa to 1500 kDa, more preferably from 600 kDa to 1000 kDa, most preferably from 700 kDa to 800 kDa. In a specific embodiment the CMC may have a molecular weight of about 725 kDa.

In a preferred embodiment the hydrogel has a viscosity from 1000 mPa*s to 9000 mPa*s at 25°C, more preferably from 2000 mPa*s to 6000 mPa*s, most preferably from 2500 mPa*s to 4500 mPa*s at 25°C. Preferably the hydrogel characterized by the afore described viscosity has a CMC concentration of 1% w/w.

In a highly preferred embodiment, the hydrogel may be characterized by a thixotropic behavior. These thixotropic properties are characterized by an increased shear stress of the gel at an increases shear rate and a decreased shear stress in response to a decreasing shear rate. The hydrogel may be characterized by a shear stress of between about 50 Pa to about 350 Pa, about 60 Pa to about 300 Pa, about 70 Pa to about 250 Pa, or about 80 Pa to about 230 Pa at a shear rate of about 600 1/s. Preferably the hydrogel may be characterized by a shear stress of between about 150 Pa to about 250 Pa, more preferably 180 Pa to about 220 Pa, and most preferably of about 200 Pa at a shear rate of about 600 1/s. Preferably the hydrogel may be characterized by a shear stress of below about 350 Pa, below about 300 Pa, below about 250 Pa, or below about 220 Pa at a shear rate of about 600 1/s. The respective thixotropic property advantageously allows shear thinning during the spraying process of the gel and a subsequent rearrangement of the hydrogel structure and its viscous properties on top of the skin.

The hydrogel comprising an antibody may further be characterized by a viscosity of about 500 mPa*s to about 1000 mPa*s, more preferably about 600 mPa*s to about 800 mPa*s at about 32°C at an antibody concentration of about 10 mg/ml to about 15 mg/ml, most preferably at an antibody concentration of about 12,5 mg/ml and/or a viscosity about 1250 mPa*s to about 2000 mPa*s, more preferably about 1400 mPa*s to about 1700 mPa*s at about 8°C and a concentration of the incorporated antibody of about 10 mg/ml to about 15 mg/ml, most preferably at a concentration of the antibody of about 12,5 mg/ml.

The hydrogels according to the present invention may preferably be stable for at least 2 months, more preferably more than 4 months and most preferably for more than 6 months under sterile conditions. According to one aspect of the stability, the viscosity characteristics as described afore are maintained over the respective period of time.

The pressing forces required to initiate the spraying of the hydrogel of the present invention through a nozzle depend on the concentration of the gelling agent, the type of gelling agent comprised in the hydrogel and the nozzle. Preferably, the forces required to initiate the spraying of the hydrogel of the present invention are between about 10 and 35 N, preferably between 20 and 35 N 30 N and 32 N, more preferably between 30 N and 31 N. Preferably a Ursatec 3K spraying device (Ursatec Verpackung GmbH, St. Wendel, Germany) or similar spray devices, which are well known to the person skilled in the art, may be used for spraying the formulation according to the invention.

The inventive hydrogel formulation advantageously remains on the area of application, such as the skin, or a wound or other topical body site, due to a higher viscosity and adhesion, while other hand being sprayable to allow very convenient, pain free application of a well defined dose. A dose can be well defined by using a dose pump and such dose pumps work well within a certain corridor of viscosity. Furthermore a higher, thixotropic viscosity allows to adjust the formulation in a way, that even when applying a spray nozzle and the pertaining spray force, the spray is not finely dispersed into very small droplets that can diffuse away from the target area but forms larger droplets that impact on the target body site an finally form a confluent film layer of the gelled formulation.

As antibodies are predominantly reactive in their monomeric form, a further aspect of stability of a hydrogel comprising an antibody is characterized by the dimerization or aggregation of the antibodies in the gel. A stable composition maintains monomeric antibodies in high amounts and exhibits only a minor extend of dimerization or even aggregation of the antibodies. The hydrogel according to the present invention comprises more than about 80%, preferably more than about 85% of monomeric antibodies for at least about 5 weeks, preferably 11 weeks and most preferably least about 6 months upon storage at 2°C to 8°C. The hydrogel according to the present invention comprises less than about 10% w/w preferably less than about 5% w/w of aggregated antibodies after at least about 5 weeks, preferably about 11 weeks and most preferably about 6 months upon storage at 2°C to 8°C. The dimerization and aggregation of an antibody may be determined by size exclusion chromatography as generally known in the art.

In summary, the hydrogels according to the present invention thus surprisingly combine thixotropic characteristics which provide a good sprayability with a high viscosity in order to achieve maximal adhesiveness on the skin, the possibility to allow constant dosing and a high stability.

The hydrogel may comprise 2 mg to 50 mg, 4 mg to 20 mg, or preferably 5 mg to 15 mg of antibody per gram hydrogel. Most preferably, the hydrogel comprises about 10 mg of 15 mg antibody per gram hydrogel.

The hydrogel may further comprise an alcohol, preferably a polyol, such as glycerol or propylene glycol. The hydrogel may further preferably comprise at least one buffering agent to maintain a dermatologically acceptable pH. Generally the hydrogel may be buffered to have a pH in a range of about 3 to about 8 or about 6 to about 8. The buffering agent may be any of those already known in the art as useful in preparing medical formulations, for example 20 mM phosphate buffer, pH 7.4. Further suitable buffers include, but are not limited to, acetic acid/acetate buffers; hydrochloric acid/citrate buffers; citrate-phosphate buffers; phosphate buffers; citric acid/citrate buffers; lactic acid buffers; tartaric acid buffers; malic acid buffers; glycine/HCl buffers; saline buffers such as phosphate buffered saline (PBS), Tris-buffered saline (TBS), Tris-HCl, NaCl, Tween buffered saline (TNT). Self buffered formulations are another embodiments of the inventions, where no buffer is added and the drug protein itself may act as a pH stabilizing component.

Furthermore, the hydrogel may comprise at least one antioxidant or other preservatives already known in the art as useful in preparing medical formulations.

The hydrogel according to the present invention may be obtained by mixing the required amount of gelling agent with a buffered aqueous solution. The mixture may be agitated to dissolve the gelling agent. Furthermore, the mixture might be heated to promote the solution of the gelling agent. Preferably the solution may be heated to between about 30°C and about 50°C, more preferably to between about 35°C and about 45°C. The solution may also be cooled to improve the gelation further. The obtained hydrogel may subsequently be sterilized, preferably by heat or irradiation. For example the hydrogel may be steam sterilized at about 121°C and about 2 bar for 15 min.

The hydrogel according to the present invention may be obtained by producing a stock hydrogel comprising a concentration of gelling agent which is higher than the concentration of gelling agent in the final hydrogel composition comprising the antibody. After the stock hydrogel is prepared as described afore, the stock hydrogel is diluted to the appropriate final concentration with a sterile antibody solution. In an alternative embodiment, the hydrogel may be obtained by mixing the gelling agent and/or a solution comprising the gelling agent with an antibody solution before sterilization. In this embodiment the subsequent sterilization may preferably be performed by irradiation.

The administration of the polyclonal antibody and/or the compositions described herein may be effected locally or systemically by different ways, e.g. by intravenous, intraperitoneal, subcutaneous, intradermal, intramuscular, topical, intranasal or intrabronchial administration. Preferably, the antibody or composition is administered topically on the skin or mucosa.

The dosage regime may be determined by the attending physician based on clinical factors. For systemic infections or diseases, the polyclonal antibody and/or the composition is preferably administered systemically, preferably intravenously or intraperitoneally. For a localized infection or disease, the antibody and/or the composition is preferably administered locally, most preferably by a topical or intradermal administration. The polyclonal antibody and/or the composition may also be administered locally and systemically at the same time. Most preferably, the antibody and/or the composition is administered topically. The antibody may also be administered orally in form of a mouth rinse or by gurgling. Further to topical administration forms like cream, ointment, or gel as described above, the antibody may be administered topically in form of eye drops. In the formulation for the application in the eye, gel formers or thickeners like cellulose derivatives, hyaluronic acid and other hydrophilic gel formers as described above may also be applied, eventually at similar or lower concentrations as for topical gels for the skin. I the case of ointments for the eye, such formulations may have the same or higher viscosity as gels for topical use on the skin.

The present invention also encompasses the administration of the antibody disclosed herein and/or the pharmaceutical composition to a subject in need thereof. It is generally known that dosages for anyone patient depend upon many factors, including the patient's size, body surface and area, age, the particular compound to be administered, sex, time and route of administration, general health or other drugs being administered concurrently. Progress can be monitored by period assessment during the administration. For repeated administration over several days or longer, depending on the condition to be treated, the treatment is sustained until a desired suppression of the disease or the symptoms occurs.

The antibody according to the present invention may be administered as a monotherapy or together with at least one other active agent. The other active agent(s) may be administered separately or as a part of a pharmaceutical composition of the present invention together with the polyclonal antibody. Preferably, the other active agent(s) is an antibacterial agent, most preferably an antibiotic agent as described above.

Thus, in a further aspect, the pharmaceutical composition according to the present invention may comprise at least one other active agent in addition to the antibody. Preferably, the other active agent is an antibacterial agent, an anti-inflammatory agent, phage or a phage enzyme. Most preferably an antibiotic agent as described above.

The anti-inflammatory agent may be a non-steroidal-anti-inflammatory-agent or preferably a steroid. The steroid may be selected from the hydrocortisone types, an acetonide, a betamethasone type, a halogenated steroid ester or a labile steroid prodrug. The hydrocortisone type steroid may be selected from hydrocortisone, methylprednisolone, prednisolone, prednisone, and triamcinolone. The acetonide may be selected from amcinonide, budesonide, desonide, fluocinolone acetonide, fluocinonide, halcinonide, and triamcinolone acetonide. The betamethasone type steroid may be selected from beclometasone, betamethasone, dexamethasone, fluocortolone, halometasone, and mometasone. The halogenated steroid ester ester may be selected from alclometasone dipropionate, betamethasone dipropionate, betamethasone valerate, clobetasol propionate, clobetasone butyrate, fluprednidene acetate, and mometasone furoate. The labile steroid prodrug may be selected from ciclesonide, cortisone acetate, hydrocortisone aceponate, hydrocortisone acetate, hydrocortisone buteprate, hydrocortisone butyrate, hydrocortisone valerate, prednicarbate, and tixocortol pivalate. Preferably the steroid is a hydrocortisone type steroid, more preferably prednisolone.

In still a further embodiment, the invention relates to a method of treating or preventing infections of *Staphylococcus* genus bacteria and/or *Staphylococcus* genus bacteria-related diseases. The method comprises a step of administering an antibody or a pharmaceutical composition as described herein to a subject in need thereof. The antibody or the pharmaceutical composition is administered to the subject in a therapeutically active amount. Preferably, the *Staphylococcus* is from the *S. aureus,* most preferably from the *S. intermedius* group of bacteria. Preferably, the antibody or pharmaceutical composition is administered topically.

In another embodiment, the invention relates to a medicament comprising the antibody as described herein.

In a further embodiment, the invention relates to the use of an antibody as described above in the manufacture of a medicament for the treatment of for treating and/or preventing infections of *Staphylococcus* genus bacteria and/or *Staphylococcus* genus bacteria -related diseases.

In yet another embodiment, the present invention relates to a method of manufacturing a medicament for the use in preventing infections of *Staphylococcus* genus bacteria and/or *Staphylococcus* genus bacteria-related diseases , comprising the steps of:
(a) obtaining a polyclonal antibody from a naturally occurring antibody source ,
(b) optionally purifying or partially purifying the polyclonal antibody by chromatography techniques.

Within this embodiment, step (a) of obtaining the polyclonal antibody from a naturally occurring antibody source may be performed as described above. Within this embodiment, step (b) of purifying or partially purifying the polyclonal antibody by chromatography techniques may be performed as described above. The method for manufacturing may also comprise a step of preparing a pharmaceutical composition comprising the polyclonal antibody.

In an alternative embodiment, the invention relates to a method of inhibiting the proliferation of *Staphylococcus* bacteria, preferably *S*. *aureus,* most preferably from the *S*. *intermedius* group of bacteria, wherein the method comprises at least the step of exposing the bacteria to an antibody as described above.

In a further aspect, the invention relates to a polyclonal anti-*S*. *aureus* alpha-hemolysin protein antibody (also termed anti-*S*. *aureus* Hla protein antibody) for use in a method for treating or preventing infections of *S*. *intermedius* group bacteria and/or *S. intermedius* group bacteria-related diseases.

The polyclonal anti-*S*. *aureus* alpha-hemolysin protein antibody may be comprised in a pharmaceutical composition or in a medicament as described afore.

A polyclonal "anti-*S*. *aureus* Hla protein antibody " or "anti-*S*. *aureus* Hla antibody" is considered to be an antibody that specifically binds to an epitope within an *S. aureus* Hla protein, wherein "specifically binding" means that the antibody may not bind in a significant percentage to a protein which is not an *S. aureus* Hla protein at the same antibody concentration and the same amount of antigenic protein. In some embodiments, a specific binding may avoid binding of a protein which is not a *S. aureus* Hla protein greater than 15%, 10%, 9%, 8%, 7%, 6%, 5%, 4%, 3%, 2%, 1% or 0.5%. In a further embodiment, an antibody which specifically binds to an epitope binds with a binding constant which is below 100 nM, preferable below 10 nM, even more preferred below 1 nM. In addition to antibody molecules that specifically bind to an epitope within an *S. aureus* Hla protein, a "polyclonal anti-S. *aureus* Hla protein antibody" may also comprise antibody molecules that do not bind to an epitope within an *S. aureus* Hla protein. Preferably, a polyclonal anti-*S*. *aureus* Hla antibody comprises at least 1%, more preferably at least 2%, most preferably at least 2.5% of antibody molecules that specifically bind to an epitope within an *S. aureus* Hla protein.

The *S. aureus* Hla protein according to the invention may also be a fragment, a portion, variant, derivative or mutant of the sequence according to UniProtKB/Swiss-Prot Database Entry No. P09616.2. Such mutants may comprise one or more deletions or one or more conservative amino acid substitutions in the primary sequence. A "derivative" may be a polypeptide according to UniProtKB/Swiss-Prot Database Entry No. P09616.2 which is chemically and/or enzymatically derivatized at one or more amino acids, including side chain modifications, backbone modifications and N- and C-terminal modifications including acetylation, methylation, hydroxylation, amidation, phosphorylation and the attachment of carbohydrate or the lipid moieties, cofactors, and the like.

In a highly preferred embodiment, the *S. aureus* Hla protein is a mutant form of *S. aureus* Hla according to UniProtKB/Swiss-Prot Database Entry No. P09616.2 which has reduced hemolytic activity. More preferably, the mutant is devoid of hemolytic activity. Within the context of the present invention, a protein is considered "devoid of hemolytic activity", when it does not possess a hemolytic activity in a hemolytic activity determination assay as published in Menzies and Kernodle, 1994. The publication Menzies and Kernodle, 1994, discloses mutant forms of the polypeptide according to amino acids 27 to 319 of the UniProtKB/Swiss-Prot Database Entry No. P09616.2 wherein the histidine in position 35, 48, 144 or 259 are substituted by leucine (mutants H35L, H48L, H144L and H259L). Mutants H48L, H144L and H259L exhibit a reduced hemolytic activity of 7, 16 and 46% and may be used as an *S. aureus* Hla protein according to the present invention. In a highly preferred embodiment of the present invention, the *S. aureus* Hla protein is a polypeptide according to SEQ ID NO: 1 which comprises a substitution of histidine 35 against leucine (H35L). According to Menzies and Kernodle, 1994, this mutant is devoid of hemolytic activity.

The polyclonal anti-S. *aureus* Hla antibody may be obtained by immunizing an animal with an *S. aureus* Hla protein as described herein. An antibody is considered to be obtained by immunizing an animal with an *S. aureus* Hla protein, when the step of immunizing the animal is comprised in the production of the polyclonal *S. aureus* Hla antibody. The production of the antibody obtained by immunizing an animal may of course comprise additional steps, for example to isolate and/or purify the antibody. The polyclonal anti-S. *aureus* Hla antibody may, for example, be obtained by immunizing rodents, mammals, especially ungulates, avians. The mammal may be a mouse, rat, sheep, dog, goat, rabbit, equine or bovine. Preferably, the antibody is obtained by immunizing a sheep, goat, rabbit, equine or bovine, most preferably a bovine. For immunization an animal with an *S. aureus* Hla protein according to the present invention, said protein is administered to said animal.

Obtaining a polyclonal antibody by immunizing an animal with an *S. aureus* Hla protein is especially advantageous, since it represents a fast and relatively inexpensive process for obtaining antibodies.

The *S. aureus* Hla protein used for obtaining the polyclonal anti-S. *aureus* Hla antibody may preferably be a mutant form of *S. aureus* Hla comprising an H35L mutation as described above. For immunization, the *S. aureus* Hla protein may be used in form of a fusion protein comprising a carrier polypeptide and one or more *S. aureus* Hla proteins *S. aureus* Hla proteins may be a carrier protein itself. The carrier polypeptide may be antigenic, stimulating the immune system to react to the fusion protein, thereby generating an immune response in an organism. The antigenic carrier polypeptide may be a keyhole limpet hemocyanin (KLH), concholepas hemocyanin (CCH), bovine serum albumin (BSA), ovalbumin (OVA). Fusions with carrier proteins may especially be used when only short *S. aureus* Hla protein fragments of SEQ ID NO: 1 are used.

In an alternative embodiment, the animal may be immunized by administering a polynucleotide vaccine. The polynucleotide vaccine may comprise a polynucleotide, preferably a DNA fragment, encoding an *S. aureus* Hla protein described herein. The polynucleotide may furthermore comprise regulatory elements, for example a promoter, that regulate the transcription of the polynucleotide sequence encoding the *S. aureus* Hla protein. The promoter may be inducible or a constitutive promoter. The constitutive promoter may, for example, be the early cytomegalo virus (CMV) promoter.

The protein or polynucleotide for immunizing an animal with an *S. aureus* Hla protein may be administered by any suitable route. Preferably, the protein or polynucleotide may be administered orally, intranasally or by intraperitoneal or intramuscular injection or injection into a mammary gland or a supramammary lymph node. In a highly preferred embodiment, the protein for immunizing the animal is administered by subcutaneous injection.

In a highly preferred embodiment, an *S. aureus* Hla protein comprising a H35L mutation is administered to a bovine by subcutaneous injection.

The protein or polynucleotide for immunizing an animal with an *S. aureus* Hla protein may be administered in combination with one or more adjuvants, particularly as an immunostimulatory substance. The adjuvant may be selected based on the method of administration and may include mineral oil-based adjuvants, such as Freund's complete and incomplete adjuvant, Montanide incomplete Seppic adjuvant, such as ISA, especially Montanide ISA51, oil-in-water emulsion adjuvants, such as Ribi Adjuvant System, syntax adjuvant formulations containing muramyl dipeptide or aluminium salt adjuvants. In a preferred embodiment, the adjuvant is a saponin adjuvant, more preferably, a saponin adjuvant derived from *Quillaja saponaria,* most preferably the adjuvant is Quil-A (Invitrogen, San Diego, USA).

The protein or polynucleotide for immunizing an animal with an *S. aureus* Hla protein may be administered once or several times. Generally, immunizing schedules may be determined by the person skilled in the art depending on the *S. aureus* Hla protein, the animal species used and the amount of protein or nucleotide used for immunization. Preferably, the protein is administered in an initial priming administration and additionally in further boost administration in intervals of 5 to 20 days, preferably 7 to 14 days. More preferably, a first boost administration is administered 5 to 10 days after the priming administration and a second boost administration is administered 16 to 26 days after the priming administration. Most preferably, a first boost administration is administered 7 days after the priming administration and a second boost administration is administered 21 days after priming administration. Generally, further boost administrations of protein may be administered, until a desired titer of *S. aureus* Hla antibodies is determined in the blood of the immunized animal.

The protein or polynucleotide for immunizing an animal with an *S. aureus* Hla protein may be administered in any suitable amount required for generating an antibody response to the S. *aureus* Hla protein in the respective animal. The *S. aureus* Hla protein may be administered in an amount of 0.001 µg to 100 µg per kg body weight, preferably 0.01 µg to 10 µg per kg body weight, and more preferably 0.1 µg to 1 µg per kg body weight, and most preferably 0.3 µg to 0.7 µg per kg body weight of the immunized animal.

The polyclonal antibodies, uses, compositions and methods for manufacturing according to the present invention solve the problem underlying the invention. The antibodies and compositions according to the invention advantageously provide an effective treatment for *S. intermedius* group bacterial infections and related diseases, especially for infections and related diseases of *S. pseudintermedius.* The treatment based on described polyclonal antibodies is advantageously a non-antibiotic treatment and is thus also suitable for the treatment and prevention of infections and diseases related to drug-resistant bacteria.

Notably, the polyclonal antibodies may easily be obtained since it is not required to immunize animals with an isolated antigen. Since the antibodies may be obtained from animals not immunized with isolated *Staphylococcus* genus bacteria, isolated part thereof, an isolated *Staphylococcus* genus bacteria protein, and/or isolated part or fraction thereof, large amounts of material, such as milk or colostrum, is available. Thus, the costs for producing the polyclonal antibodies and respective compositions comprising the antibodies is low. While low manufacturing costs are generally advantageous, low costs are especially advantageous in the production of medicaments for pets.

Advantageously, the antibodies and compositions according to the invention exert their therapeutic effect upon topical administration. Systemic antibacterial antibody treatments, like passive immunization, require the administration of the medication by a medical professional, such as a veterinarian or a medical doctor. In contrast thereto, the topically administered antibodies and compositions of the present invention can be conveniently and repeatedly administered by the owner of an animal or a human patient himself. Also, immunological reactions against the administered antibody, leading to side effects or diminishing the therapeutic effect, are less likely to occur upon topical administration of an antibody. Furthermore, due to the limited body surface usually treated by a topical treatment, smaller amounts of antibodies are required as compared to systemic treatment.

In a further aspect, the invention generally relates to an antibody that binds to at least one epitope selected from epitopes comprising the amino acid sequence KIGGLIG, ATKQQSN, KKILVIRTK, IDVIYERV, KAADNFLDP and/or DSDINIK. Most preferably the antibody binds to at least the epitope comprising the amino acid sequence KIGGLIG. Preferably the antibody is an immunoglobulin G that binds to at least one epitope selected from epitopes comprising the amino acid sequence KIGGLIG, ATKQQSN, KKILVIRTK, and/or IDVIYERV. In another preferred embodiment the antibody is an immunoglobulin A or M that binds to at least one epitope selected from epitopes comprising the amino acid sequence IDVIYERV, KAADNFLDP. The antibody according to this aspect may further be characterized by the properties and characteristics as described above.

The present invention shall be explained in more detail by the following figures and examples.

### Figures

Fig. 1 shows a chromatogram of an affinity chromatography of a polyclonal anti *S.aureus* Hla antibody on a *S.aureus* Hla H35L column.
Fig. 2 shows the binding of polyclonal anti *S.aureus* Hla antibody samples to *S.aureus* Hla H35L in an ELISA assay.
Fig. 3A shows the inhibition of Hla induced lysis of rabbit red blood cells by polyclonal antibodies obtained from an immunized cow before and after formulation.
Fig 3B and 3C, show the lysis of rabbit red blood cells induced by supernatants of *S. pseudintermedius* strains cultures 69687 (Fig. 3B) and 4639949 (Fig. 3C) and the inhibition of lysis by different IgG preparations
Fig. 4 A) and B) show the FACS diagrams of the binding of polyclonal anti S.aureus Hla antibodies to the surface of different *S. pseudintermedius* strains.
Fig. 5 shows the penetration of a polyclonal anti S.aureus Hla antibody into piglet skin. A) Fluorenscence stain of intact skin. b) HE stain of laserporated skin. c) Fluorenscence stain of laserporated skin.
Fig. 6 shows a rheogramm of hydrogels with different CMC concentrations.
Fig. 7 shows the spray forces required to activate the spray process of different CMC concentration from an Ursatec 3K horizontal spray system.
Fig. 8 shows viscosities of placebo and antibody containing hydrogels at storage temperature (8°C) and skin temperature (32°C) directly after preparation and two or six months after storage.
Fig. 9 shows relative content of monomer, dimer and larger aggregates as well as recovery of plgGs in CMCs hydrogels of two different batches directly after preparation and after storage.
Fig. 10 shows CD spectra of antibodies in PBS and hydrogel before and after storage for 7 weeks at 2°C to 8°C.

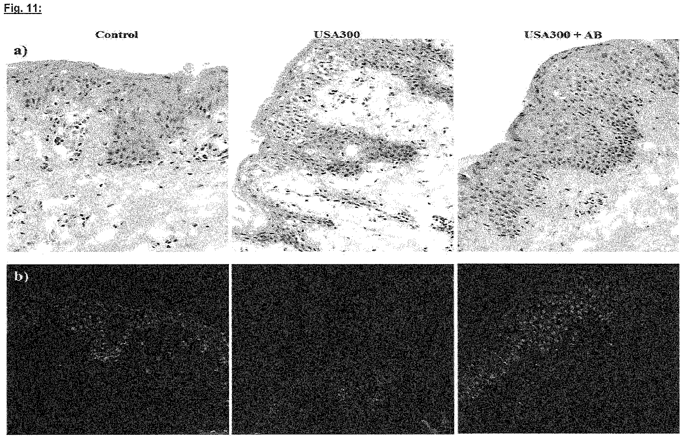

Fig. 11 shows the effect of S. aureus and anti-AT antibody on morphology and cell viability of human skin.
Fig. 12 shows a microarray analysis for pre-staining and secondary antibodies of Example 10. A) Pre-staining, 1:2000; B) Adjusted scan; C) Anti-bovine IgG (H+L) DyLight680 (1:2000), anti-bovine IgG (Fc) DyLight800 (1:2000)
Fig. 13 shows a microarray of sample A of Example 10. A) Sample A, 10 µg/ml (IgG); B) Sample A, 10 µg/ml (Ig); C) Sample A (polyclonal antibody pool before immunization), 10 µg/ml
Fig. 14 shows a microarray of sample B of Example 10. A) Sample B, 10 µg/ml (IgG); B) Sample B, 10 µg/ml (Ig); C) Sample B (polyclonal antibody pool before immunization), 10 µg/ml
Fig. 15 shows a microarray of sample C of Example 10. A) Sample C, 10 µg/ml (IgG); B) Sample C, 10 µg/ml (Ig); C) Sample C (polyclonal antibody pool before immunization), 10 µg/ml

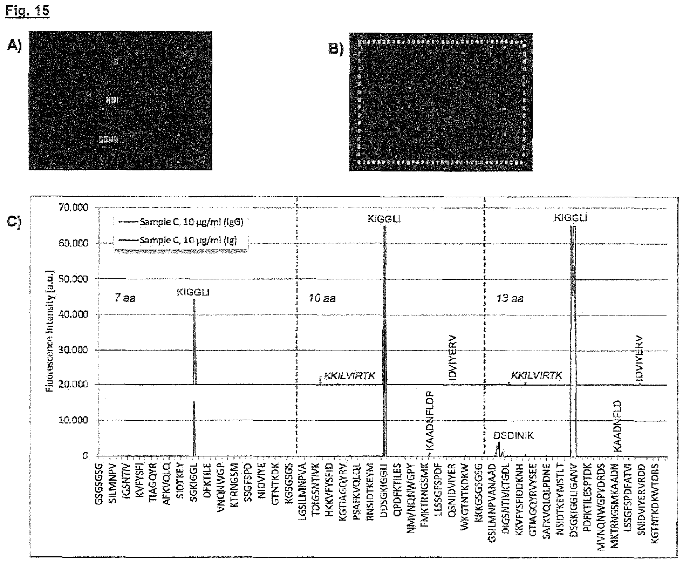

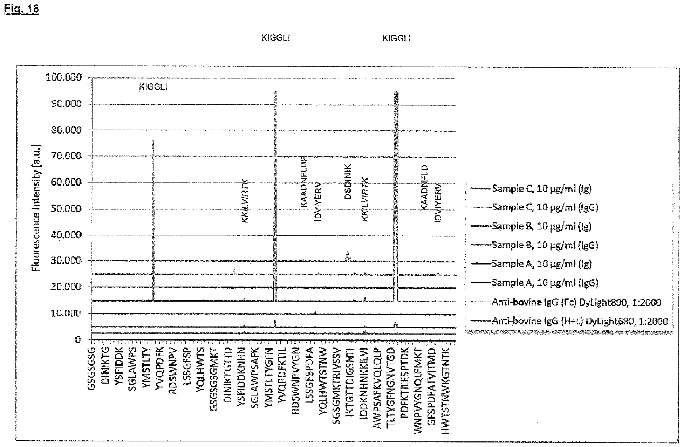

Fig. 15 shows a comparison of the microarray results

### Examples

### Example 1: Isolation and purification of IgG

### 1.1 Collection of milk

Milk was collected over 7 days after delivery. The collected milk was stored at about -20°C.

The milk was either obtained from cows that were not immunized with an isolated antigen preparation or from cows immunized with *aureus* Hla H35 protein.

For immunization, a pregnant cow was immunized with 300µg of recombinant mature *S. aureus* Hla H35L protein in PBS with Quil-A (Invitrogen, San Diego, USA) as adjuvant. *S. aureus* Hla comprising a H35L mutation was obtained by cloning, expressing in *E. coli* and purification as disclosed in Menzies and Kernodle, 1994 and Wardenburg & Schneewind 2008. In total, a volume of 2ml was injected subcutaneously. The priming immunization was administered on day 0 and two boost immunizations were administered on days 14 and 21. The cow delivered on day 42.

### 1.2 Milk processing

The frozen milk was thawn and defatted at 50°C in a disk separator at 8,000 g to a fat content of less than 0,1% w/w according to commonly known methods.

The defatted milk was subjected to 7 cycles of diafiltration with a microfiltration membrane of 0.14µm pore size and a utrafiltration membrane with a cut-off size of 10kD at a constant pressure difference (ΔpTM) of 2 bar. During this step casein micelles, bacteria and small molecular compounds were separated.

The whey obtained from diafiltration was stored at about -20°C.

### 1.3 Capture-Chromatography

To capture the polyclonal antibodies from the whey fraction the whey was thawn over 12h at room temperature.

The whey (20 g/l Immunglobulin) was adjusted to 20 mM NatriumKaliumphosphatbuffer pH 7,5; 250 mM NaCl and loaded on a Capto-MMC column (GE Healthcare Bio-Sciences, Pittsburgh, USA) with a bed volume of 10l (40 cm x 8 cm). The flow through comprised the polyclonal antibodies whereas proteins like lactoperoxidase and lactoferrin were bound to the column. The collected flow through was subsequently loaded on a MEP HyperCel column (Pall Corporation, New York, USA) with a bed volume of 30 l (40 cm x 24 cm). The column was washed with 5 column volumes of Buffer A (20 mM NaKCO3 pH 7,5; 250 mM NaCl). Subsequently, pre-elution was performed with 2 column volumes of buffer B (50 mm MES/NaOH pH 6,0). Subsequently the bound Ig was eluted with 3 column volumes of buffer C (50 mM Na acetate pH 4,). MEP HyperCel column chromatography was performed at a flow rate of 5 l/min. The IgG comprising elution fractions were pooled.

Alternatively, the whey was subjected to Sepharose Q chromatography to separate beta-Lactoglobulin from the whey. In brief, one volume of whey was diluted with three volumes of Na-PFA-Buffer (30 mM Na₂HPO₄, 30 mM Na-formiat, 60 mM Na-acetate, pH 5.5). The diluted whey was loaded on a HiTrap Q FF (GE Healthcare) column at a flowrate of 1 ml/min. The flow-through comprising the IgG was further processed, whereas beta-Lactoglobulin was bound to the column.

A buffer exchange was performed by dialyzing the pooled fractions against PBS (20 mM Na2HPO4 pH 7,5; 150 mM NaCl) over a PES Membrane (100 kD cutoff) at 7°C. Subsequently, the pooled polyclonal anti *S. aureus* Hla H35L IgG fractions were sterile filtered over a Millipore Express SHC 0,5/0,2 µm double membrane and frozen at -20°C.

### Example 2: Hydrogel formulation

### 2.1. Formulation

2,5% w/w of Sodium Carboxymethylcellulose (CMC) Blanose® 7H4F (Ashland, Kentucky, USA) was added to PBS pH 7.4 (137 mM NaCl, 2,7 mM KCI, 9,0 mM Na2HPO4 · 2 H2O, 3,5 mM KH2PO4) under vigorous stirring. The mixture was heated to 40°C and stirred over 24h. Afterwards, the hydrogel was steam sterilized (121 °C, 2 bar, 15 min).

Thawed polyclonal anti *S. aureus* Hla H35L IgG as obtained in Example 2 with a protein concentration of 40-80 mg/ml was added to the CMC stock solution under stirring over 5 to 10 min at room temperature to obtain a hydrogel with a CMC concentration of 1,5% w/w and a protein concentration of 10 to 12 mg/g.

### 2.2. Analysis of hydrogel formulation

### 2.2.1. Rheological behaviour

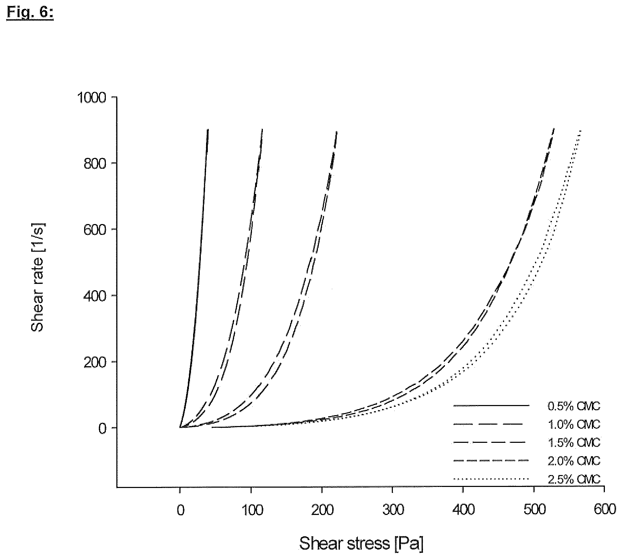

Gel characteristics of hydrogels comprising 0%, 0,5%, 1%, 1,5%, 2% and 2,5% w/w CMC were determined with a MCR 100 rheometer (Anton Paar, Graz, Austria) with a stainless steel plate/plate system (diameter 25 mm). Viscosity measurements were performed in oscillation mode with a constant deformation of 0.5% and a constant angular frequency of 10 s⁻¹. The samples were equilibrated to a temperature of 8°C (storage temperature) and 32°C (skin temperature), respectively. The rheological properties of hydrogels were measured in rotation mode with a logarithmic increase in shear rate from 1-900 s⁻¹ followed by a logarithmic decrease in shear rate from 900-10 s⁻¹. The rheological properties investigated at a room temperature of 20°C as well as at storage temperature of 8°C. The respective rheogramms are shown in Fig. 6. By an increasing shear rate the shear stress increases, whereas with a decreasing shear rate the process is reversed. The hysteresis loop, the area enclosed by the up and down curve, is a typical characteristic for thixotropic behavior. This behavior of the hydrogel allows shear thinning during the spraying process and afterwards a rearrangement of the hydrogel structure and its viscous properties on top of the skin.

### 2.2.2 Spraying forces

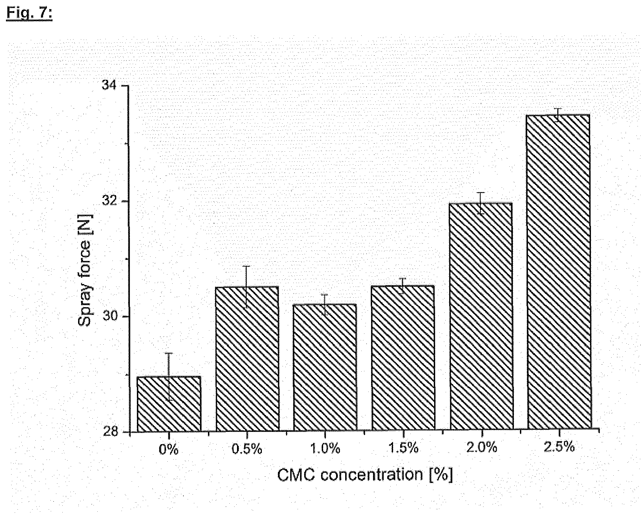

Spraying forces were tested using a TA.XT *Plus* Texture Analyser (Stable Micro Systems). A stainless steel plate was attached to the machine and used to compress the Ursatec 3K spray pump and determine their force needed to release the gel. The results for hydrogels having CMC concentrations of 0%, 0,5%, 1%, 1,5%, 2% and 2,5% CMC are shown in Fig. 7. Data are presented as mean ± SD (n = 3).

### 2.2.3 Viscosity

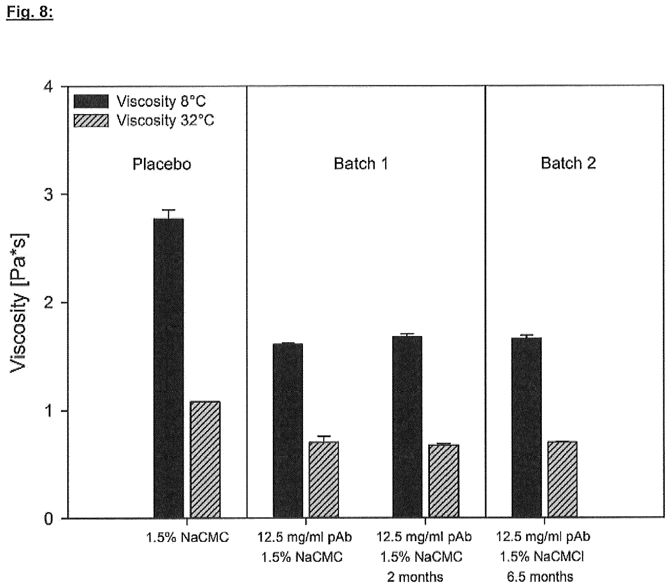

The viscosity at 8°C and 32°C of a hydrogel formulation comprising 1,5% w/w CMC and 12,5 mg/ml polyclonal antibody obtained as described was determined after preparation and after storage for 2 and 6,5 months. For comparison, the viscosity of a placebo formulation without antibody was determined. The results are show n in Fig. 8. Data is presented as mean ± SD (n = 3).

### 2.2.4 SEC analysis

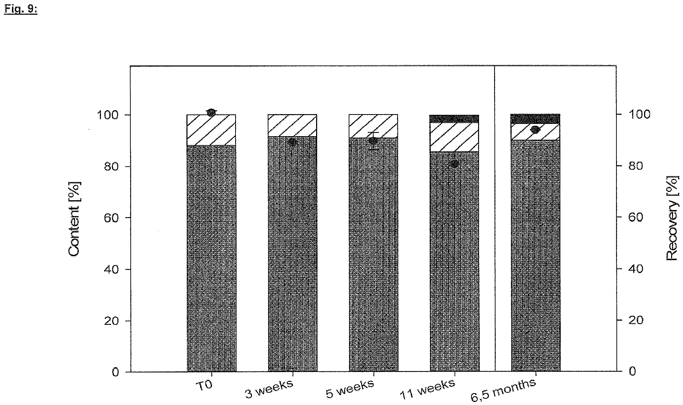

The potential aggregation was evaluated by size exclusion chromatography (SEC). SEC analytics was conducted on a Waters 2695 system (Waters GmbH, Eschborn, Germany). The flow rate of the running buffer (50 mM phosphate, 300 mM NaCl, pH 7.0) was 0.5 ml/min and 25 µl of each sample was injected onto an YMC Pack-diol 300 column and detected with UV-detection at 280 nm. Protein concentrations were determined by SEC using a standard curve. The results are shown in Fig. 9. Aggregation in a freshly prepared batch of hydrogel ,as prepared according to 2.1, was determined at to and after storage for 3, 5 and 11 weeks at 2°C to 8°C, and for a second hydrogel batch after storage for 6,5 months. The results are shown in Fig. 9.

The relative amount of dimers only slightly varies over time. Only a minor amount of aggregated was observed even after > 6 months of storage.

From this data it can be concluded that the secondary structure of the plgG is stable in the gel formulation at the concentration desired for in vivo studies (10 mg/g).

### 2.2.4. Circular dichroism (CD) analysis

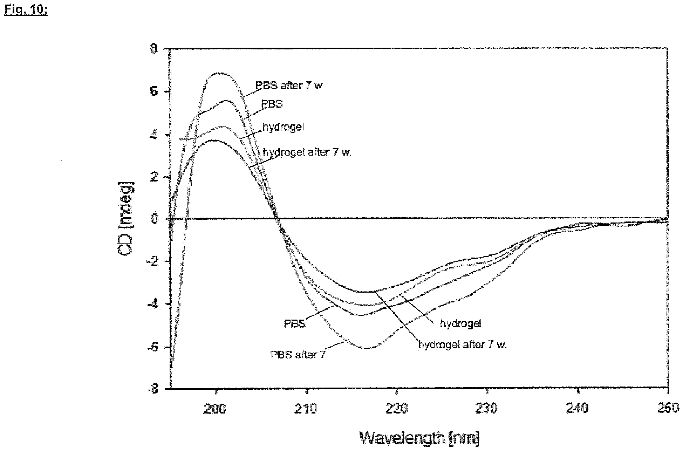

Secondary structure is an important hallmark for activity of an antibody. Prevention of secondary structure in the hydrogel compared to PBS after preparation and upon storage for 7 weeks at 2°C to 8°C was analysed. The far-UV CD region (180-240 nm) corresponds to the peptide bond absorption and gives information on the secondary structure of a protein. For far-UV CD, the antibody hydrogel formulations were diluted to concentrations between 0.159 mg/ml and 0.209 mg/ml with phosphate buffered saline pH 7.4 and measured with a Jasco J-715 spectropolarimeter (Jasco International, Tokyo, Japan) in a quartz cuvette with a path length of 0.1 cm at 20°C. Far-UV CD spectra were collected in a continuous scanning method from 190 to 250 nm at a scanning speed of 50 nm/min, a response time of 1 s, a bandwidth of 1 nm, steps of 0.1 nm and an accumulation of 4 scans. Using the Spectra Analysis Software (Jasco International, Tokyo, Japan), the spectra were background corrected for the spectrum of the respective buffer or placebo gel and curves were smoothed. Data is recorded in millidegrees of ellipticity as a function of wavelength. The resulting spectra are shown in Fig. 10. Data shows the far-UV spectra of the antibodies in PBS directly after preparation as described above and after seven weeks of storage at 2-8°C and the respective samples formulated in 1.5% CMC gel.

The spectra show typical curves of immunoglobulins representing high beta-sheet content. This can be concluded from the negative maximum ∼ 217 nm, a zero ellipticity at ∼ 210 nm and a positive maximum - 200 nm. Differences in the amplitudes are caused by variation in the concentrations and the heterogeneity of antibody batches.

It can be concluded that there are no changes in secondary structure of the plgG upon storage in PBS pH 7.4 or 1.5% CMC gel.

From CD and the SEC data it can be concluded that the secondary structure of the antibodies according to the invention are stable in the gel formulation according to the invention.

### Example 3. Affinity chromatography

### 3.1 Column preparation

A 1 ml affinity column comprising S. aureus Hla H35L prepared as follows. HiTrap NHS-activated HP Sepharose (GE Healthcare), 1 ml; with 10 µmol NHS/ml Sepharose was washed with 6 ml of cold 1 mM HCI. Subsequently, the column was equilibrated twice and 1 ml recombinant alpha-hemolysin H35L protein solution (1 mg/ml in PBS) was added and incubated for 15 min at RT. Subsequently, the column was subjected to sequential washing with, 1 ml PBS, 6 ml 0.5 M Ethanolamine (pH 8.1, 0.5 M NaCl), 6 ml 0.1 M Natriumacetatebuffer (pH 4.0, 0.5 M NaCl), 6 ml 0.5 M Ethanolamine (pH 8.1, 0.5 M NaCl) and incubated for 15 min at RT. The sequential washing was repeated with a final washing step with 6 ml PBS. All flow rates were about 1 ml/min. Before chromatography, the column was equilibrated with 20 ml of Buffer A (0.1 M Glycin/Tris pH 8.0).

Sample preparation: 1.6 ml of polyclonal S. aureus Hla H35L antibody obtained in Example 1 (75 mg/ml protein in PBS) were diluted with 10.4 ml Buffer A.

### 3.2 Chromatography

Chromatography was performed at a flowrate of 1 ml/min. 10 ml of the sample solution were loaded on the affinity column. The flow through during loading was collected in fraction 1. Subsequently, the column was washed with 10 ml of Buffer A. The flow through during washing was collected in fraction 2. Elution was subsequently performed with Buffer B (0.1 M Glycin/HCl pH 2.7, 0.5 M NaCl). The eluate was collected in samples 3 to 12 in 1ml fractions. Protein concentration of the flow through was monitored as absorbance at 280 nm. The two peak fractions 5 and 6 were pooled. 15 µl 1.5 M Tris/HCl pH 8.8 was added to adjust the pH 7. The samples were concentrated with an Amicon Ultra-2, 100K (Millipore) cell by centrifugation over 5 min at 4800 rpm, Rotor S4180 (Beckman).

### 3.3 Result

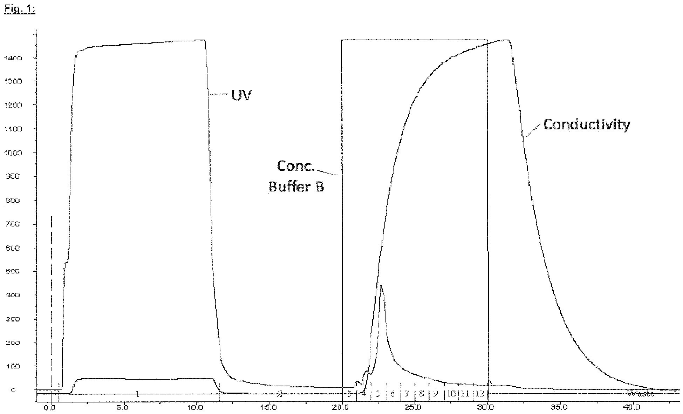

The chromatogram is shown in Fig. 1. It can be concluded from the relation of the AUC at 280 nm of the flow through during loading and the AUC of the elution fraction of the affinity column that the polyclonal antibody sample comprised about 3% of polyclonal IgG which specifically binds to the *S. aureus* Hla H35L protein.

### Example 4: Determination of toxin binding by ELISA

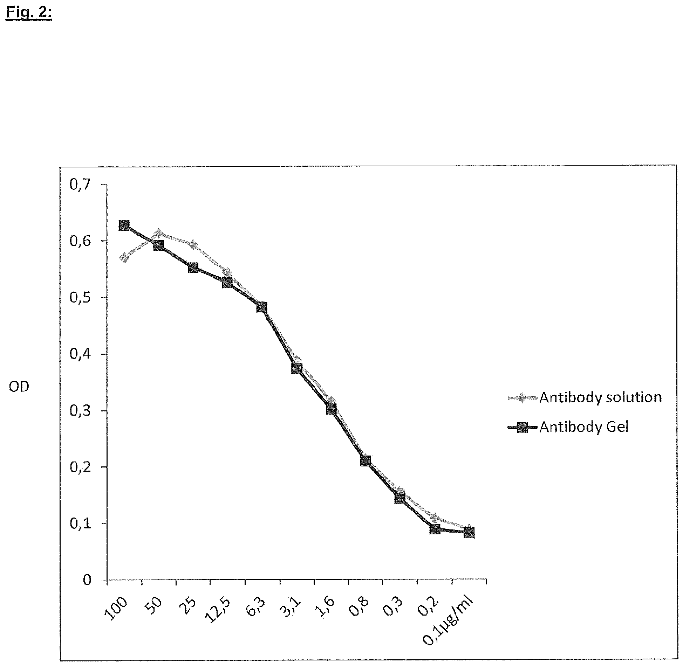

96-wells plates were coated with 0.3 µg/ml recombinant *S. aureus* Hla. After blocking with BSA and washing with PBS with 0.05% Tween-20 the purified polyclonal anti *S. aureus* Hla IgG pool obtained from an immunized cow according to Example 1 (see. Fig 2 "Antibody solution) was added to the plate *S*. *aureus* starting with 100 µg/ml and diluted in 1:2 dilution steps. Detection was done with a peroxidase conjugate Goat-anti-bovine Ig antibody (Jackson Immunoresearch). TMB was used as substrate and the reaction was stopped with H2SO4 (1N). Absorbance was measured at 450 nm. The assay was repeated with antibodies from a hydrogel formulation as obtained in Example 2.1 (see Fig.2 "Antibody Gel").

As shown in Fig. 2, the polyclonal *S. aureus* Hla IgG exhibits a concentration dependent binding to the recombinant *S. aureus* Hla.

The ELISA assay was repeated with S. aureus. beta-hemolysin, leukotoxins LukA, LukB, LukC , LukD , LukE, LukS, gamma-hemolysin components A (HIgA) and B (HIgB). The results for the ID50 values obtained in theses assays is summarized in Table 1.

**Table 1: Toxin binding of purified polyclonal antibody**

| **Toxin** | **ID50 in µg/ml** |
|---|---|
| alpha-toxin | 3 |
| beta-toxin | 25 |
| LukA | 0 |
| LukB | 0 |
| LukD | 25 |
| LukE | 50 |
| LukF | 25 |
| LukS | 0 |
| HIgA | 0 |
| HIgB | 25 |

Surprisingly, the IgG pool obtained from a cow immunized with alpha-hemolysin did not only comprise significant amounts of anti-alpha-hemolysin antibodies, but also antibodies reactive to beta-hemolysin, LukD, LukE, LukF, and HlgB.

### Example 6: Neutralization of Hla dependent lysis of rabbit red blood cells

### 6.1 Inhibition of recombinant alpha-hernolysin induced lysis

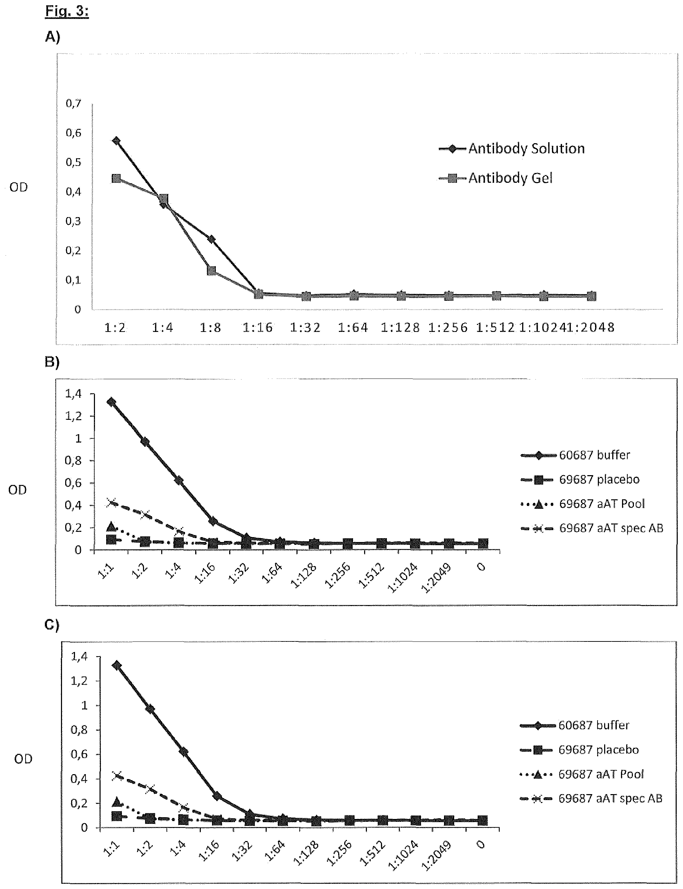

The purified IgG obtained from a cow immunized with *S. aureus* Hla H35L as obtained in Example 1 and 2 was analyzed in a red blood cell-based neutralization assay. The ability of polyclonal anti *S. aureus* Hla IgG (PlgGs) before (Fig. 3A, "Antibody Solution") and after formulation (Fig. 3A, "Antibody Gel") to lyse rabbit erythrocytes (RBC) was tested in a 96-well format. Specifically, 140 µl from each antibody sample was loaded into the first well and then serially diluted 2-fold, up to 1: 2048. After the dilution of each sample, 140 µl of Hla (10ng/ml) was added to each well incubated at RT for 2 h. 5x10⁶ rabbit RBC in 1× PBS was added to each well and following incubation at RT for 2h, plates were centrifuged for 5 min, 100 µl of the supernatant was removed gently to a new microtiter plate, and absorbance was read at 415 nm.

As shown in Fig. 3A, the polyclonal anti *S. aureus* Hla IgG (PlgGs) exhibit a concentration dependent inhibition of RBC. Based on the Hla concentration in the assay and the amount of added polyclonal IgG, it can be concluded 37,5 % of the anti *S. aureus* Hla IgG (PlgGs) are neutralizing.

### 6.2 Inhibition of S. pseudintermedius supernatant induced lysis

The purified IgG from a cow which was not immunized with an isolated antigen (natural IgG pool) was compared with the purified IgG from a cow immunized with *S. aureus* Hla H35L (aAT pool) obtained in Example 1 and Hla H35L affinity purified IgG obtained in Example 3 (aAT spec AB) in a red blood cell-based neutralization assay as described above in 6.1. In contrast to the assay of 6.1, culture supernatants from cultures of S. *pseudintermedius* strains 69687 and 4639949 were used instead of recombinant Hla for induction of lysis.

As shown in Fig. 3A and 3B, the IgG from a cow which was not immunized with an isolated antigen (natural IgG pool) and the purified IgG from a cow immunized with *S. aureus* Hla H35L (aAT pool) surprisingly show a similar inhibition of *S. pseudintermedius* induced cell lysis, whereas the inhibition by Hla H35L affinity purified IgG obtained in Example 3 (aAT spec AB) exhibited a lower inhibition of cell lysis. Thus, the inhibition of hemolysis is only partially dependent on the anti Hla activity of the polyclonal antibodies.

### Example 7: antibody binding to drug resistant S. pseudintermedius strains

For surface staining of *S. pseudintermedius* with polyclonal antibody derived in Example 1, the bacteria were diluted to 5 x 107 c/ml, in 15 µl per well (OD0,5=5x108 bacteria/ml) and Ab dilutions were prepared in PBS (0,1 BSA) to a 2x final concentration.
15 µl antibody solution were added to 15 µl bacteria solution and incubated for 30 min at 4°C and shaking at 750 rpm 170 µl. PBS buffer were added to wash. After centrifugation 7 min at 3500 rpm, supernatants were taken off. Subsequently, 25 µl of staining solution comprising anti-bovine IgG Alexa Fluor 647 1/350 (Jackson) was added and samples were incubated for 30 min at 4°C and shaking at 750 rpm. The samples were washed with 200 µl buffer. After centrifugation 7 min at 3500 rpm, supernatants were taken off pellets were fixed with 150 µl 1% PFA (1/10 x from stock 10%). Subsequently, the FACS measurement was performed. Table 2 shows the *S. pseudintermedius* analyzed:

**Table 2: Drug resistant S. pseudintermedius strains**

| Strain | Source | Phenotye | Resistance Profile |
|---|---|---|---|
| 23939 | Ireland/2008/skin | DR-MRSP ST68 SCC mecV(T) | OXA-PEN-AMP-LEX-KAN-ERY-CLI-TET-SXT-CIP |
| 69687 | UK/2012/skin | MDR-MRSP ST71 SCCmec11-111 | OXA-PEN-AMP-AMC-LEX-GEN-KAN-ERY-CLI-SXT-CI P |
| MRSPHH15 | Germany/2012/ski | MDR-MRSP ST71 SCCmec11-111 | OXA-PEN-AMP-AMC-LEX-GEN-KAN-ERY-CLI-TET -SXT-CIP |
| GL151A | Germany/2012/wound | MDR-MRSP ST71 SCCmec11-111 | AMP-AMC-LEX-GEN-KAN-ERY-CLI-TET-SXT-CIP |
| BNG I | UK/2011/skin | MRSP ST260 SCCmecV | OXA-PEN-AMP-LEX-TET |
| GL118B | Germany/2012/skin | MDR-MSSP ST262 | PEN-AMP-KAN-TET |
| 4639949 | USA/2012/skin | MSSP ST309 | PEN-AMP-TET |
| GL117B | Germany/2012/ear | MDR-MSSP ST263 | PEN-AMP-KAN-ERY |

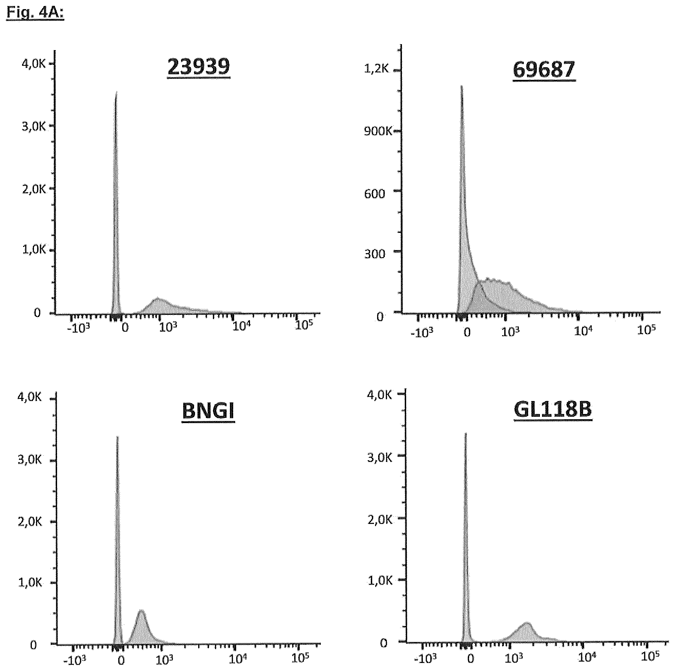

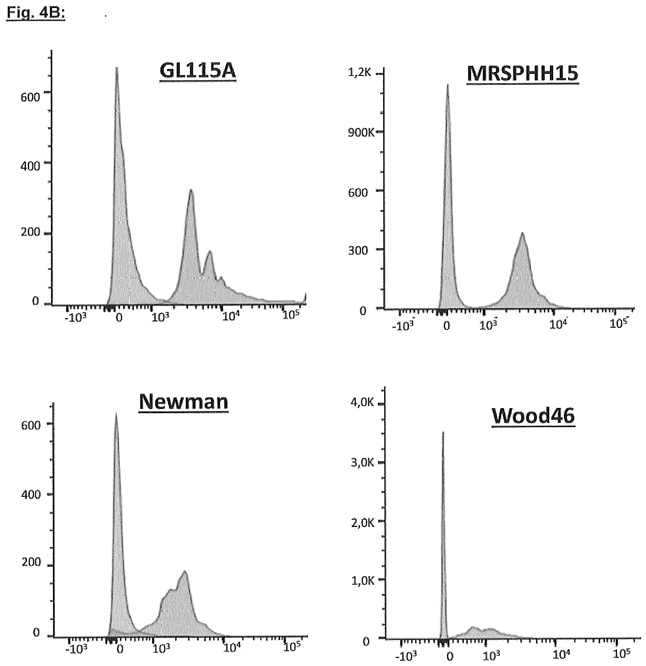

Result: The anti-Hla antibody binds on the surface of various s. pseudintermedius strains in a concentration dependent manner. The resulting FACS diagrams are depicted in Fig. 4A and B.

### Example 8: In vivo administration on piglet skin and human skin

### 8.1 Piglet skin

### 8.1.1. Material and Methods:

A polyclonal anti- *S. aureus* Hla antibody as obtained in Example 2 was biotinylated and formulated in a hydrogel in accordance with Example 3. The hydrogel was applied in explanted piglet skin. Prior to application of the hydrogel, a part of the skin sample was laser porated with a P.L.E.A.S.E device (Pantec Biosolution, Ruggell, Liechtenstein) in a depth of 107µm.
The skin was subsequently cultivated over 24 h in a Franz-Cell. After 24h The skin was cryo-conserved and sliced in a cryotome. The biotinylated antibody was visualized with streptavidin-Alexa Fluor 488. Subsequently, skin slides were examined by confocal laser microscopy.

### 8.1.2. Results:

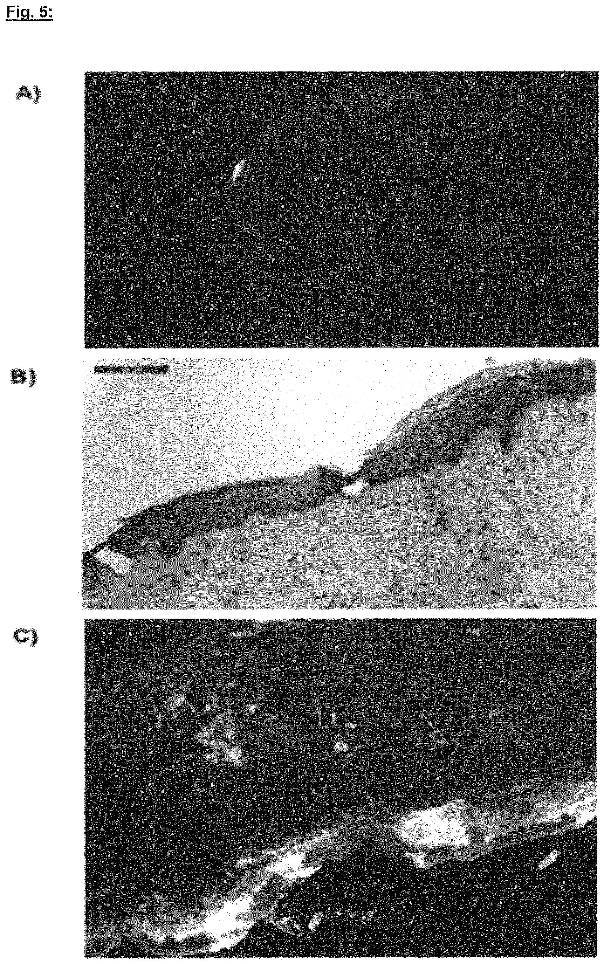

As shown the antibody comprised in the administered hydrogel does not penetrate intact skin and is localized on the skin surface (Fig. 5A). In the laser-porated parts of the skin, the antibody showns penetration and distribution into the dermis (Fig. 5C). Figure 3B shows the skin morphology by HE stain.

### 8.2. Human skin

The effect of the polyclonal antibody according to the invention on the structure of epidermis and epidermal barrier in human skin sections colonialized with the methicillin-resistant *Staphylococcus aureus* strain USA300 was investigated. After colonization of S. aureus USA300 on 8mm biopsies of normal human skin and co-application of 10mg/ml polyclonal IgG as described above in PBS, the skin was cultivated over 24 h in a transwel system in a CO2 incubator. Afterwards the biopsies were frozen at -140 and kryosections were stained to investigate the morphology. The results are shown in Fig. 10. Fig 10 a) shows a H&E stain of skin sections to investigate the morphology of epidermis and dermis, Magnification: 20x Fig 10 b) shows an immunofluorescence staining of E-cadherin on skin sections to make damage on cell-cell junctions visible. In green: goat-anti-mouse E-Cadherin + donkey-anti goat Alexa Fluor 488. In red: TO-PRO®-3 nucleic acid (nucleus) counterstain. Scale bar: 50 µm Magnification

### Example 9: Clinical trial

A clinical study was performed to assess the effect of the polyclonal antibody formulation obtained according to Example 2 for the treatment of intertrigo and pyotraumatic dermatitis in dogs. The study was performed according to the following protocol:

### 9.1 Study Protocol

### Introduction

The aim of this study is to evaluate the use of anti-polyclonal *S. aureus* Hla antibodies in the treatment of two disorders, namely intertrigo (part 1) and pyotraumatic dermatitis (part 2).

### Study protocol:

### Study design-Part 1:

This is a study using polyclonal anti-polyclonal *S. aureus* Hla antibodies for the treatment of intertrigo in the dog. Owners will sign an informed consent form (Appendix 1) prior to inclusion in the study.

### Inclusion criteria:

Twenty dogs with intertrigo will be included in this study. Intertrigo will be diagnosed by history, clinical examination and cytology of impression smears of the folds showing neutrophils and numerous coccal bacteria present.

### Exclusion criteria:

Dogs will be withdrawn from the study if there is no improvement within the first two weeks after inclusion. Furthermore, dogs will be excluded with any severe adverse effects associated with the treatment. Lastly, a lack of owner compliance will be considered a reason for exclusion.

### Intervention:

All owners will be asked to fill out a questionnaire regarding the clinical history and development of Intertrigo of the dog. Before start of the treatment blood is taken to analyze pre-existing antibodies against *Staphylococcus.* The intertrigo lesions will be treated with a polyclonal anti- *S. aureus* Hla antibody as obtained in Example 3 twice daily. A clinician not involved in the treatment will judge the improvement clinically and cytologically. Improvement will be judged for clinical signs of erythema and exudate between 0 and 3 (0 - normal; 1-Mild; 2 - Moderate; 3 - Severe), cytology results for the treated and control area for cocci, rods and yeast between 0 and 4 (0= No bacteria/yeast/inflammatory cells; 1= Occasional bacteria / yeast / inflammatory cells present, but slide must be scanned carefully for detection; 2= Bacteria / yeast/ inflammatory cells present in low numbers, but detectable rapidly without difficulties; 3= Bacteria/yeast/inflammatory cells present in larger numbers and detectable rapidly without any difficulties; 4= Massive amounts of bacteria/yeast/ inflammatory cells present and detectable rapidly without difficulties).

### Clinical evaluation:

Photographs will be taken from each affected area and labeled with intertrigo_ownername_dogname_date.jpg. Cutaneous cytology will be obtained from each affected area at each visit. The cytology specimens will be air dried and stained with Diff Quick. They will be evaluated by a veterinarian not involved in the treatment in a blinded fashion using a previously reported semiquantitative scale (Budach et al 2012).

### Study design-Part 2:

This is a study using polyclonal anti-polyclonal *S. aureus* Hla antibodies for the treatment of pyotraumatic dermatitis in the dog. Owners will sign an informed consent form prior to inclusion in the study.

### Inclusion criteria:

Ten dogs with pyotraumatic dermatitis will be included in the study. The condition will be diagnosed by history, clinical examination and cytology of the lesion showing neutrophils and coccal bacteria.
Medications to treat other concurrent diseases such as flea allergy dermatitis can be continued during the study.

### Exclusion criteria:

Dogs will be withdrawn from the study if there is no improvement within the first two weeks after inclusion. Furthermore, dogs will be excluded with any severe adverse effects associated with the treatment. Lastly, a lack of owner compliance will be considered a reason for exclusion.

### Intervention:

All owners will be asked to fill out a questionnaire regarding the clinical history and development of hot spot of the dog. The affected area will be clipped with a sterilized no #40 clipper blade and cleaned with an antiseptic. All dogs will be treated with prednisolon at 0.5-1 mg/kg daily for three days and all dogs will be treated twice daily with anti-polyclonal *S. aureus* Hla in a hydrogel spray comprising 10mg/ml polyclonal anti *S. aureus* Hla as obtained in Example 3 for 14 days. After the treatment period is finished blood will be taken to analyze anti-staphloccocus antibodies.

### Clinical evaluation:

Photographs will be taken from the lesion and labeled with podo_ownername_dogname_date.jpg. Cutaneous cytology will be obtained from the lesion prior to inclusion and after two weeks. The cytology specimen will be air dried and stained with Diff Quick. They will be evaluated in a blinded fashion by a veterinarian using a previously reported semiquantitative scale (Budach et al 2012)).

### 9.2. Results

The first dog with hot spot was included in the study showed an improvement of symptoms and no side effects after treatment.

### Example 10: Conformational Epitope Mappings of Polyclonal Antibody

### 10.1. Material and Methods

Microarray Content: The sequence of Staphylococcus aureus alpha toxin was elongated by neutral GSGSGSG linkers at the C- and N-terminus to avoid truncated peptides. The elongated antigen sequence was translated into linear 7, 10 and 13 amino acid peptides with peptide-peptide overlaps of 6, 9 and 12 amino acids. Afterpeptide synthesis, all peptides were cyclized via a thioether linkage between a C-terminal cysteine and an appropriately modified N-terminus. The resulting conformational alpha toxin peptide microarrays
contained 963 different peptides printed in duplicate (1,926 peptide spots), and were framed by additional HA (YPYDVPDYAG, 98 spots) control peptides.

| | |
|---|---|
| Samples: | Polyclonal antibody obtained from cows not immunized with isolated antigen according to Example 2 (Sample A); polyclonal antibody obtained from cows immunized with aureus Hla H35 protein; affinity purified polyclonal antibody obtained according to Example 3 (Sample C). |
| Washing Buffer: | PBS, pH 7.4 with 0.005% Tween 20 (2 x 10 sec after each assay) |
| Blocking Buffer: | Rockland blocking buffer MB-070 (30 min before the first assay) |
| Incubation Buffer: | Washing buffer with 10% blocking buffer |
| Assay Conditions: | Antibody concentration of 10 µg/ml in incubation buffer; incubation for 16 h at 4°C and shaking at 140 rpm |
| Secondary Antibodies: | Rabbit anti-bovine IgG (Fc) DyLight800 (1:2000) and anti-bovine IgG (H+L) DyLight680 (1:2000); 45 min staining in incubation buffer at RT |
| Control Antibody: | Mouse monoclonal anti-HA (12CA5) DyLight680 (1:2000) and mouse monoclonal anti-HA (12CA5) DyLight800 (1:2000); 45 min staining in incubation buffer at RT |
| Scanner: | LI-COR Odyssey Imaging System; scanning offset 0.65 mm, resolution 21 µm, scanning intensities of 7/7 (red = 680 nm/green = 800 nm) |

Pre-staining of a conformational alpha toxin peptide microarray was performed with secondary rabbit anti-bovine IgG (Fc) DyLight800 antibody (1:2000) for IgG analysis or with secondary anti-bovine IgG (H+L) DyLight680 antibody (1:2000) and with control mouse monoclonal anti-HA (12CA5) DyLight800 antibody (1:2000) for total Ig analysis to investigate background interactions with the cyclic constrained antigen-derived peptides that could interfere with the main assays. Subsequent incubation of other alpha toxin peptide microarrays with the polyclonal antibody samples A, B and C at a concentration of 10 µg/ml in incubation buffer was followed by staining with the secondary and control antibodies as well as read-out at scanning intensities of 7/7 (red/green). The additional HA peptides framing the peptide microarrays were simultaneously (rabbit anti-bovine IgG (Fc) DyLight800 antibody) or subsequently (anti-bovine IgG (H+L) DyLight680 antibody) stained as internal quality control to confirm the assay quality and the peptide microarray integrity. Quantification of spot intensities and peptide annotation were based on the 16-bit gray scale tiff files at scanning intensities of 7/7 that exhibit a higher dynamic range than the 24-bit colorized tiff files. Microarray image analysis was performed with PepSlide® Analyzer. A software algorithm broke down fluorescence intensities of each spot into raw, foreground and background signal, and calculated averaged median foreground intensities and spot-to-spot deviations of spot duplicates. Based on averaged median foreground intensities, an intensity map was generated and interactions in the peptide map highlighted by an intensity color code with red for high and white for low spot intensities. A maximum spot-to-spot deviation of 40% was tolerated, otherwise the corresponding intensity value was zeroed.
Additionally, averaged spot intensities of the assays with the antibody samples were plotted against the antigen sequence from the N- to the C-terminus of Staphylococcus aureus alpha toxin was to visualize overall spot intensities and signal-to-noise ratios. The intensity plots were correlated with peptide and intensity maps as well as with visual inspection of the microarray scans to identify epitopes that were recognized by the antibody samples. In case it was not clear if a certain amino acid contributed to antibody binding, the corresponding letters were written in gray. For a better data overview, the baselines of the intensity plots were leveled.

### 10.2 Anti-bovine IgG (H+L) DyLight680 (1:2000), anti-bovine IgG (Fc) DyLight800 (1:2000)

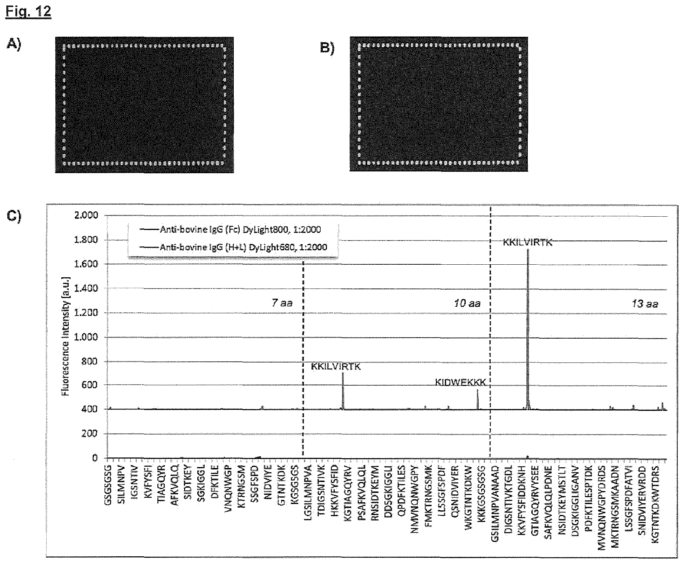

The alpha toxin peptide microarray was incubated with (1) secondary rabbit anti-bovine IgG (Fc) DyLight800 antibody (1:2000) and (2) with secondary anti-bovine IgG (H+L) DyLight680 antibody (1:2000) and control mouse monoclonal anti-HA (12CA5) DyLight800 antibody (1:2000) was followed by read-out at a scanning intensities of 7/7 (red/green).As shown in Figure 12, no background interaction of secondary anti-bovine IgG (H+L) DyLight680 antibody in the red channel at 700 nm; weak background interactions of secondary rabbit anti-bovine IgG (Fc) DyLight800 antibody with basic peptides with the consensus motifs KKILVIRTK and KIDWEKKK in the green channel at 800 nm was observed. A well-defined staining of HA control peptides was observed.

### 10.3. Sample A (polyclonal antibody pool before immunization), 10 µg/ml

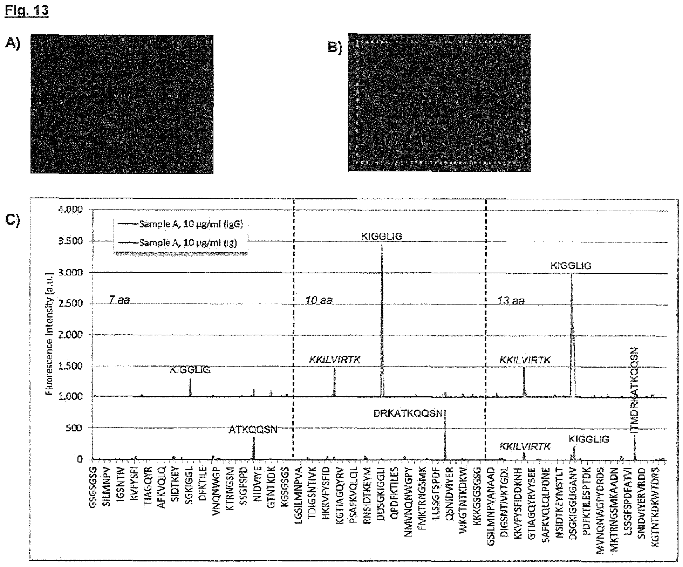

The alpha toxin peptide microarray was incubated with sample A at a concentration of 10 µg/ml was followed by staining with the secondary and control antibodies as well as read-out at a scanning intensities of 7/7 (red/green). As shown in Figure 13, a weak IgG response against a epitope-like spot patterns formed by adjacent peptides with the consensus motifs KIGGLIG with all peptide lengths, weak background interaction of the secondary antibody with peptides with the basic KKILVIRTK; additional weak and presumably less specific non-IgG response against peptides with the C-terminal ATKQQSN motif was observed. A moderate signal-to-noise ratios was observed. Also, a well-defined subsequent staining of HA control peptides was observed.

### 10.4 Sample B (polyclonal antibody pool after alpha toxin immunization). 10 µg/ml

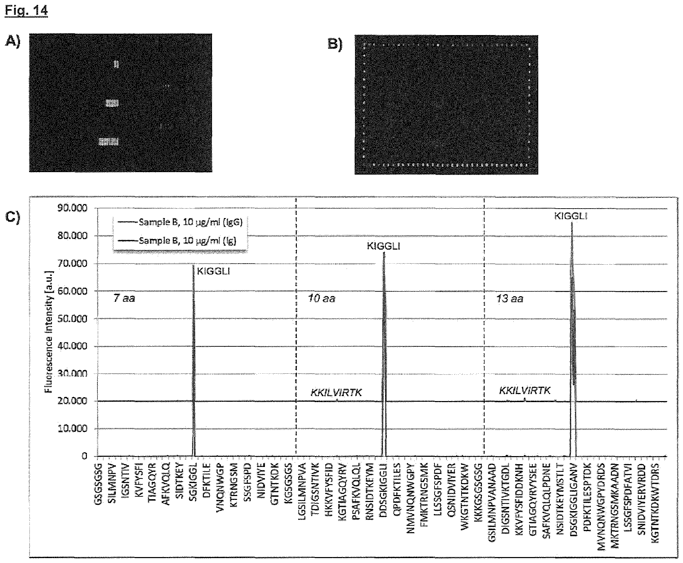

The alpha toxin peptide microarray was incubated with sample B at a concentration of 10 µg/ml was followed by staining with the secondary and control antibodies as well as read-out at a scanning intensities of 7/7 (red/green). As shown in Figure 14, a very strong IgG response against a epitope-like spot patterns formed by adjacent peptides with the consensus motifs KIGGLI with all peptide lengths, weak background interaction of the secondary antibody with peptides with the basic KKILVIRTK motif; no additional non-IgG response was observed. Also, a high signal-to-noise ratios and a well-defined subsequent staining of HA control peptides was observed.

### 10.5 Sample C (polyclonal antibody pool after alpha toxin immunization), 10 µg/ml

The alpha toxin peptide microarray was incubated with sample C at a concentration of 10 µg/ml was followed by staining with the secondary and control antibodies as well as read-out at a scanning intensities of 7/7 (red/green). As shown in Figure 15, a very strong IgG response against peptides with the consensus motifs KIGGLI with all peptide lengths, weak additional IgG response against peptides with the IDVIYERV motif, weak background interaction of the secondary antibody with peptides with the basic *KKILVIRTK;* additional weak non-IgG responses against peptides with the consensus motifs KAADNFLDP and DSDINIK was observed. Also, a high signal-to-noise ratios and well-defined subsequent staining of HA control peptides was observed.

### 10.6 Summary of results

The PEPperMAP® Conformational Epitope Mappings of polyclonal antibodies obtained from cows not immunized with isolated antigen according to Example 2 (Sample A), polyclonal antibodies obtained from cows immunized with S. aureus Hla H35L protein and affinity purified polyclonal antibodies obtained according to Example 3 (Sample C) were performed against alpha toxin of Staphylococcus aureus translated into cyclic constrained 7, 10 and 13 amino acid peptides with peptide-peptide overlaps of 6, 9 and 12 amino acids. The corresponding alpha toxin peptide microarrays were incubated with the antibody samples at a concentration of 10 µg/ml in incubation buffer followed by staining with secondary and control antibodies as well as read-out with a LI-COR Odyssey Imaging System. Quantification of spot intensities and peptide annotation were done with PepSlide® Analyzer. Pre-staining of an alpha toxin peptide microarray with secondary rabbit anti-bovine IgG (Fc) DyLight800 antibody or with secondary anti-bovine IgG (H+L) DyLight680 antibody and control mouse monoclonal anti-HA (12CA5) DyLight800 antibody highlighted very weak background interaction of the secondary rabbit anti-bovine IgG (Fc) DyLight800 antibody with basic peptides with the consensus motifs KKILVIRTK and KIDWEKKK. Incubation of the antibody samples resulted in the following observations:
Sample A (polyclonal antibodies obtained from cows not immunized with isolated antigen) showed a weak IgG response against peptides with the consensus motif KIGGLIG, albeit with ∼20 fold lower spot intensities and signal-to-noise ratios compared to samples B and C; moreover, we observed a weak and presumably less specific non-IgG response against peptides with the C-terminal ATKQQSN motif
Sample B (polyclonal antibodies obtained from cows immunized with aureus Hla H35 protein) showed a very strong IgG response against peptides with the consensus motif KIGGLI; otherwise we only observed a weak background interaction of the secondary rabbit anti-bovine IgG (Fc) DyLight800 antibody with peptides with the basic consensus motif KKILVIRTK; additional IgA or IgM responses were not identified
Sample C (affinity purified polyclonal S. aureus Hla H35 antibodies) also showed a very strong IgG response against peptides with the consensus motif KIGGLI as well as a weaker IgG response against peptides with the consensus motif IDVIYERV; moreover, we observed additional weak IgA or IgM responses against peptides with the consensus motifs KAADNFLDP and DSDINIK.

### References

Bannoehr J, Guardabassi L. Staphylococcus pseudintermedius in the dog: taxonomy, diagnostics, ecology, epidemiology and pathogenicity. Vet Dermatol. 2012, 23(4):253-66.
Beco L, Guaguère E, Lorente Mendez C, Noli C, Nuttall T, Vroom M. Suggested guidelines for using systemic antimicrobials in bacterial skin infections: part 2-- antimicrobial choice, treatment regimens and compliance. Vet Rec. 2013, 172(6):156-60
Bubeck Wardenburg J, Schneewind O. Vaccine protection against Staphylococcus aureus pneumonia. J Exp Med. 2008, 18;205(2):287-94
Budach SC, Mueller RS. Reproducibility of a semiquantitative method to assess cutaneous cytology. Vet Dermatol. 2012, 23(5):426-e80.
Loeffler A, Linek M, Moodley A, Guardabassi L, Sung JM, Winkler M, Weiss R, Lloyd DH. First report of multiresistant, mecA-positive Staphylococcus intermedius in Europe: 12 cases from a veterinary dermatology referral clinic in Germany. Vet Dermatol. 2007,18(6):412-21.
Lozano C, Rezusta A, Ferrer I, Pérez-Laguna V, Zarazaga M, Ruiz-Ripa L, Revillo MJ, Torres C. Staphylococcus pseudintermedius Human Infection Cases in Spain: Dog-to-Human Transmission. Vector Borne Zoonotic Dis. 2017, 17(4):268-270.
McCarthy AJ, Harrison EM, Stanczak-Mrozek K, Leggett B, Waller A, Holmes MA, Lloyd DH, Lindsay JA, Loeffler A. Genomic insights into the rapid emergence and evolution of MDR in Staphylococcus pseudintermedius. J Antimicrob Chemother 2015; 70 (4): 997-1007.
Menzies BE, Kernodle DS. Site-directed mutagenesis of the alpha-toxin gene of Staphylococcus aureus: role of histidines in toxin activity in vitro and in a murine model. Infect Immun. 1994, 62(5):1843-7.
Piot M, Fauquant J, Madec MN, Maubois JL. Preparation of serocolostrum by membrane microfiltration. Le Lait. 2004, 84 (4):333-341.
Pomba C, Rantala M, Greko C, Baptiste KE, Catry B, van Duijkeren E, Mateus A, Moreno MA, Pyörälä S, Ružauskas M, Sanders P, Teale C, Threlfall EJ, Kunsagi Z, Torren-Edo J, Jukes H, Törneke K. Public health risk of antimicrobial resistance transfer from companion animals. J Antimicrob Chemother. 2017, 72(4):957-968.
Schmid V, Canine pyoderma Part 1: Clinical features. UK Vet. 2010, 15(8): 17 -21
Walther B, Tedin K, Lübke-Becker A. Multidrug-resistant opportunistic pathogens challenging veterinary infection control. Vet Microbiol. 2017, 200:71-78.
Zakour NLB, Bannoehr J, van den Broek AH, Thoday KL, Fitzgerald JR Complete genome sequence of the canine pathogen Staphylococcus pseudintermedius. J Bacteriol. 2011, 193(9):2363-4.

## Claims

1. A naturally occurring antibody for use in the treatment or the prevention of infections of *Staphylococcus* genus bacteria and/or a *Staphylococcus* genus bacteria-related disease.

2. The antibody of claim 1, wherein the antibody is obtained from a naturally occurring antibody source.

3. The antibody according to any of the preceding claims, wherein the naturally occurring antibody source is an animal which was not immunized with isolated *Staphylococcus* genus bacteria, isolated part thereof, an isolated *Staphylococcus* genus bacteria protein, and/or isolated part or fraction thereof.

4. The antibody according to any of the preceding claims, wherein the antibody is a polyclonal antibody.

5. The antibody according to any of the preceding claims, wherein the *Staphylococcus* is from the S. *aureus* or S. *intermedius* group of bacteria.

6. The antibody according to any of the preceding claims, wherein the antibody is immunoreactive with alpha-toxin, beta-toxin, LukD, LukE, LukF, and/or HlgB of S. *aureus.*

7. A polyclonal antibody immunoreactive with S. *aureus.* alpha-toxin, beta-toxin, LukD, LukE, LukF, and HlgB for use in the treatment or the prevention of infections of Staphylococcus genus bacteria and/or a Staphylococcus genus bacteria-related disease.

8. The polyclonal antibody according to any of the preceding claims, wherein the *Staphylococcus* genus bacterium is an antibiotic resistant strain.

9. The antibody according to claims 3 to 8, wherein the animal is a sheep, goat, rabbit, equine or bovine antibody; and/or
wherein the antibody is obtained from bovine colostrum or milk; and or
wherein the antibody is administered by topical administration

10. The antibody according to any of the preceding claims, wherein the *Staphylococcus* genus bacteria-related disease is pyoderma.

11. The antibody according to any of the preceding claims, wherein the antibody binds to at least one epitope selected from epitopes comprising the amino acid sequence KIGGLIG, ATKQQSN, KKILVIRTK, IDVIYERV, KAADNFLDP and/or DSDINIK. Most preferably the antibody may bind to at least the epitope comprising the amino acid sequence KIGGLIG.

12. The antibody according to any of the preceding claims, wherein the antibody is for treatment of infections or diseases in a dog, cat, horse or human.

13. A pharmaceutical composition comprising an antibody according to any of the preceding claims.

14. The pharmaceutical composition according to claim 13, wherein the pharmaceutical composition comprises a hydrogel.

15. An antibody that binds to at least one epitope selected from epitopes comprising the amino acid sequence KIGGLIG, ATKQQSN, KKILVIRTK, IDVIYERV, KAADNFLDP and/or DSDINIK. Most preferably the antibody may bind to at least the epitope comprising the amino acid sequence KIGGLIG.
